# EUROPEAN PATENT APPLICATION

(11) **EP 4 147 706 A1**
(43) Date of publication of application: **15.03.2023**
(21) Application number: 21799462.3
(22) Date of filing: 30.04.2021
(51) Int. Cl.: A61K 36/28, A61K 31/716, A61K 31/702, A61K 31/7016, A61K 31/733, A61P 1/16, A61P 37/02, A61P 3/02, A61P 5/38, A61P 39/00, A61K 127/00, A61K 131/00

(54) **COMPOSITION FOR REGULATING THE NEURO-IMMUNE-ENDOCRINE SYSTEM**

(30) Priority: 05.05.2020 BR 102020008884; 07.08.2020 BR 102020016156
(71) Applicant: Abou Nehmi Filho, Victor, 04644-000 São Paulo (BR); Pinhata Otoch, José, 05507-020 São Paulo (BR); Pessoa, Ana Flávia Marçal, 05545-010 São Paulo - SP (BR)
(72) Inventor: Abou Nehmi Filho, Victor, 04644-000 São Paulo (BR); Pinhata Otoch, José, 05507-020 São Paulo (BR); Pessoa, Ana Flávia Marçal, 05545-010 São Paulo - SP (BR)
(74) Representative: Linage González, Rafael
(86) International application number: PCT/BR2021/050184
(87) International publication number: WO 2021/222997

(57) **Abstract**

The present invention deals with the composition of Betaglucans and Silymarin - *Silybum marianum* and one or more Bifidogenic Prebiotics, for use as a nutritional supplement, or nutraceutical, or as a prebiotic, aiming to modulate the neuro-immune-endocrine axis, via protection and preservation of the intestinal epithelium and liver. Having as main objective to provide better quality of sleep.

The composition according to the invention comprises the following components:
- 1,3 and 1,6 beta-glucans;
- Silymarin (*Silybum marianum*)*,* or its natural or synthetic flavonolignans or flavonoids; and
- one or more Bifidogenic Prebiotics.

## Description

### Technical field

The present invention refers to the composition of 1,3 and 1,6 beta-glucans and Silymarin - *Silybum marianum* and bifidogenic prebiotics for use as a nutritional supplement, or nutraceutical, or as a prebiotic, aiming at modulating the neuro-immune-endocrine axis, recovering homeostasis, via the protection and preservation of the intestinal epithelium and of the liver. It has as main objective to provide better quality of sleep.

The composition of prebiotics and silymarin according to the invention can have a preventive and prophylactic effect for public health in relation to numerous pathologies such as viruses or Covid-19, AIDS, cancer, and other diseases such as tuberculosis, dengue, by improving the modulation of the neuro-immune-endocrine axis and protect the liver and the intestinal epithelium.

### Prior art

There is a great consensus among doctors and scientists that longevity depends on the tripod Sleep + Health + Physical Exercise. In addition, of course, to genetic and environmental factors. Interviews with super-long-lived people, over 110 years old, indicate that they all had in common, throughout their lives, a relatively stable diet, good quantity and quality of sleep, and constant but moderate physical activity. However, with the modern lifestyle it becomes increasingly difficult to maintain this tripod. Mainly, the good quality of sleep.

Good quality sleep depends on exogenous and endogenous factors. Insomnia is considered one of the great evils of the 21^{st} century and can be characterized as a state of chronic stress, which impacts immune functions and health in general. Millions of people across the planet suffer from chronic insomnia. Therefore, constant sleep deprivation has become a risk factor for the emergence, or worsening, of subsistent chronic degenerative diseases, such as obesity, diabetes, hypertension, cancer, viruses and psychic disorders (anxiety, depression).

Among the exogenous factors that can affect sleep, the discipline of time stands out, that is, it is important to sleep and wake up at the same times. It is also important to sleep in places with little or no lighting, to promote melatonin synthesis, and thermally comfortable. Melatonin produced by the pineal gland, which is associated with the hypothalamic nuclei, constitutes an important part of the neuroendocrine system, responsible for the temporal organization of the various physiological and behavioral events. However, caffeine stimulates the release of stress hormones, such as cortisol, which impairs the action of melatonin in sleep regulation along the hypothalamic-pituitary-adrenal (HPA) axis, which is the characteristic neuroendocrine hallmark of the stress response.

Among the endogenous factors that influence sleep, 3 main types can be listed, namely:
- Neurological/Emotional
- Immunological/Inflammatory
- Endocrinological/Oxidative

Neuro-emotional factors are recognized as the main causes of insomnia, but they are usually short-lived, that is, only a few days. Essentially, its mechanism of action is the lower production of serotonin in the brain during times of stress. During these periods, corticosteroids and inflammatory cytokines are released, which lead to increased peripheral and central production of kynurenine. This, in turn, is a metabolite of L-tryptophan (which is the precursor of serotonin), consequently leading to an indirect reduction in the production of serotonin in the brain. Serotonin produced in the brain is a neurotransmitter, which has the function of maintaining the balance between states of anxiety and depression. In addition, it is a precursor of melatonin, so it is essential to get more hours of sleep, that is, to avoid insomnia. It also plays a very important role in achieving deep sleep, NREM or SWS type. Bacteria of the genera *Escherichia coli, Bacillus, Ruminococcus* and *Lactobacillus* also contribute to the direct and indirect production of neurotransmitters, such as norepinephrine, dopamine, serotonin and GABA (gamma-aminobutyric acid), respectively. These bacteria, which provide positive effects on mental health, are known as psychobiotics and are part of the brain-gut communication pathway. So a stable and balanced microbiota also contributes to the modulation of the neuro-emotional system and to the improvement of sleep.

The damage that regulatory factors of the immune system can cause in sleep depends on the type of immune system that is activated. There are two types of immune systems, the innate (neutrophils, dendritic cells, macrophages, Nk - Natural Killer cells and the barriers formed by the epithelia) and the acquired (T, B and NK lymphocytes, dendritic cells). The innate and acquired immune systems work together to prevent an exacerbated inflammatory reaction, such as an anaphylactic reaction, or a chronic inflammatory reaction, such as cancer, from setting in. The imbalance between these systems caused by the release of glucocorticoids and inflammatory cytokines impairs the production of serotonin and other neurohormones, such as oxytocin, which will bring or bring harm to sleep.

Sleep can also be impaired by endocrine-hormonal factors. Generally speaking, these are oxidative processes that involve the liver and intestinal epithelium. When there are dysbiosis of the intestinal microbiota, which can be caused by drugs or changes in diet, undesirable bacteria usually adhere to the intestinal epithelium. The body's reaction is to make the intestine permeable and inject water into the place, in an attempt to physically expel the invader, causing diarrhea. However, to become permeable, there is distension of the gaps between the enterocytes (tag-junctions), which also allows the passage of bacteria and enterotoxins to the circulatory system. The immune system is then activated, which can lead to an inflammatory process if these invading bacteria are not rapidly phagocytosed by the innate immune system. Similarly, enterotoxins, upon reaching the central nervous system and the liver, are able to increase the generation of reactive species (commonly known as free radicals), resulting in oxidative stress. Consequently, in inflammatory processes, which also impair sleep.

Glucocorticoid hormones, such as corticotropin (ACTH) and cortisol, are capable of impairing the immune system, leading to immunosuppression.

Sleep neurobiology refers to the basic structural organization of normal sleep. According to it, sleep involves two states of distinct brain activity: rapid eye movement (REM) and non-REM sleep, or "Slow-Wave Sleep-SWS" sleep, which are generated in specific brain regions (NSQ).

Essentially, REM sleep, which is the period of light sleep where dreams occur, is meant for learning and memory formation. On the other hand, NREM sleep, where sleep is deeper and dreams do not occur, is a period where defective cell repairs are made and is largely responsible for the regulation of homeostasis of the body's metabolic systems.

Physiological sleep is initiated by light non-REM stages (stages 1 and 2), followed by a deeper non-REM stage (stages 3 and 4, or slow wave sleep, SWS), then REM sleep, and possibly by transient awakenings, also called pulses. In healthy young individuals, this pattern lasts approximately 90 min and is usually repeated 4-6 times a night. As the night progresses, the number and duration of arousals/pulses increase, non-REM sleep becomes more superficial, and the duration of REM episodes lengthens. It is important to note that during the different stages of life the NREM phase (SWS) decreases, making the sleep period shorter. A teenager sleeps about 10 hours a night, while an individual after age 60 sleeps between 5 and 6 hours.

It is known that sleep is one of the main determinants of growth hormone (GH) secretion and the elimination of excess cortisol. Cortisol is produced during periods of stress and has an immunosuppressive effect, and its excess in the body is eliminated during the NREM stages, mainly in NREM 3 and 4, which predominate in the first half of sleep. The elimination of excess cortisol is important to modulate the immune system, that is, to bring it back to normal by the start of the day. Otherwise, an eventual excess of immunosuppression will last throughout the day, leaving the body unprotected. And worse, if this immunosuppression process is perpetuated, it will lead to immune system dysfunction and the occurrence of autoimmune diseases, which are becoming increasingly common in the 21^{st} century. Any new cell in the body depends on the presence of this hormone. In its absence, it will increase the number of defective cells present in the body, overloading the immune system, accelerating the aging process and leading to chronic inflammation, which is harmful to sleep.

Insomnia and chronic short sleep also influence the secretion of endocrine hormones involved in metabolism. Studies show poor glucose tolerance and insulin sensitivity in sleep-restricted patients, as well as showing that reduced sleep time increases the risk of developing obesity, diabetes and cancer. Individuals who sleep 5 to 6 hours a night are at greater risk of developing these conditions than those who sleep 7 to 8 hours. Other consequences of reduced sleep time are the increase in C-Reactive Protein and the accumulation of visceral fat, all associated with the worsening of the metabolic syndrome. Likewise, sleep neurobiology shows that two important factors for chronic insomnia are dysregulation of the hypothalamic-pituitary-adrenal (HPA) axis and changes in the circadian rhythm of cortisol. In insomnia, both corticotropin-releasing hormone (CRH-produced in the hypothalamus) and cortisol (produced by the adrenal glands) are elevated. While GH (growth hormone) is decreased.

Growth hormone directly influences mitochondrial biogenesis. The lack of mitochondrial activity leads to an excess of intracellular glucose. As a result, insulin tolerance begins and a surplus of glucose in the blood, which ends up causing a series of oxidative and inflammatory reactions, before being transformed into fat. On the other hand, increased growth hormone production during NREM 3 and 4 sleep activates mitochondrial biogenesis, which consumes excess intracellular glucose. When the liver is healthy, with little inflammation and high availability of propionic acid, excess glucose not consumed by mitochondrial activity is transformed into glycogen, which also contributes to low intracellular glucose availability, making it difficult for viruses and cancer cells to multiply, and drastically reducing the formation of free radicals and the occurrence of inflammatory oxidative processes, which end up harming sleep.

Endogenous factors, which affect sleep, can be controlled by mechanisms that often act on them indirectly. Neuro-immune factors depend on the production of adequate amounts of serotonin.

Approximately 90% of the serotonin circulating in the blood is produced in the large intestine and the other 10% by the brain, in the pineal gland. Circulating serotonin is stored and transported by platelets, which are part of the immune system. Serotonin is derived from the amino acid tryptophan and uses, as cofactors, minerals such as zinc and magnesium, in addition to vitamin B6. It is important to remember that there are two groups of serotonin, one in the blood and one in the brain, which never cross. Therefore, these must be seen, from a functional point of view, as two distinct molecules. Serotonin produced in the brain acts as a neurotransmitter, while serotonin produced in the intestines acts as a hormone and regulates a wide variety of processes, both immunological and metabolic. The latter acts on the Enteric Nervous System (ENS) on intestinal peristalsis (as it has a vasoconstrictor effect on the smooth muscles of the intestines), on bone metabolism, on appetite regulation and has immunomodulatory activity, stimulating or inhibiting the proliferation of lymphocytes (Peyer's Plaque), via concentration dependent. There is evidence that the greater presence of short-chain fatty acids, preferably butyric acid, which are produced in the intestine, mainly by bacteria of the Bifidobacteria and Lactobacillum genera, stimulate the production of serotonin in the gastrointestinal tract (GIT).

These short-chain fatty acid (SFA)-producing bacteria are part of the amphibiotic microbiota and psychobiotic bacteria, which inhabit the mucus layer of the intestinal epithelium. Its function is to protect the intestinal epithelium, with the production of bacteriocins and organic acids, which maintain the balance between non-pathogenic and pathogenic species of the intestinal microbiota. In addition to lowering the pH along the gastrointestinal epithelium, which increases the absorption of minerals, due to the ionic state provided by the acidic environment. On the other hand, for the services provided, lactobacilli and bifidobacteria are not recognized by the immune system as pathogenic bacteria, but friendly to intestinal homeostasis. The population of these bacteria can be increased with the ingestion of prebiotics that have a bifidogenic effect.

Thus, maintaining a good population of lactobacilli and bifidobacteria in the intestinal epithelium contributes doubly to modulating neuro-emotional factors that affect sleep. One contribution is direct, through the neurotransmitters produced by the psychobiotic microbiota, which helps to reduce emotional stress. The other contribution is indirect, in the form of protection of the intestinal epithelium against the adhesion of pathogens and the absorption of enterotoxins. The mucus layer formed by the goblet cells, with the butyric acid produced by the bifidobacteria, works as a physical barrier to pathogens and toxins and serves as a habitat for lactobacilli, which produce the bioactive compounds and bacteriocins that fight the pathogens, preventing their adhesion and the formation of leaky gut. By preventing the installation of these inflammatory processes, the production of cortisol and glucocorticoids in the brain is reduced and, therefore, the reduction of brain serotonin levels is avoided. A good way to raise and sustain populations of lactobacilli and bifidobacteria is through the continuous intake of prebiotics, with a bifidogenic effect.

In addition to its hepato-protective effect, silymarin is a chemical compound with an antidepressant effect, as studies show that it contributes to raising the levels of noradrenaline and dopamine, as well as inhibiting the oxidative deamination of monoamines such as serotonin and dopamine. Therefore, it also contributes to the modulation of the neuro-immune-endocrine system, that is, to the improvement of sleep.

A modulated and activated immune system is the key to reducing the incidence of inflammatory factors, most often subclinical, that impair sleep. These inflammatory processes are often chronic and are a result of the modern way of life, which is very different from the way of life our ancestors evolved, along with their innate and acquired immune systems. The lower amount and quality of sleep, constant stress, sedentary lifestyle, daily diet variations and frequent travel profoundly impair the functioning of the neuro-immune system, reducing the body's defenses against toxins, endotoxins and even in the regulation of formation of unwanted cells, such as tumor cells. All this dysregulation, the result of an inflammatory process, contributes to the emergence of autoimmune diseases, such as allergies, rheumatoid arthritis, diabetes, systemic lupus erythematosus, psoriasis, Hashimoto's thyroiditis, among others. Interestingly, the emergence of such diseases is related to losses, traumas or neurological disorders, reinforcing the connection between the nervous system and other systems.

To have a functional and active immune system, which minimizes the incidence of chronic inflammation, modulation and activation is necessary. Modulation, that is, the return to normality through the elimination of excess immunosuppressive cortisol, occurs during NREM sleep, mainly in NREM stages 3 and 4, and is controlled by the availability of adequate amounts of brain serotonin. The stimulation of the immune system is done by challenges. That is, if the immune system is adapted to the constant challenges caused, whether by particles derived from food or microorganisms (viruses, bacteria, fungi) or xenobiotics, the break in the homeostasis of the endocrine neuroimmune axis is reduced and, thus, it is possible to establish or maintain the homeostasis of the organism. In the super hygienic world we live in, with a large number of vaccines, opportunities for challenges for the immune system have been missed. The alternative is to train it with substances, or components, that mimic the actions of these harmful elements, but in a harmless way, such as beta-glucans. Essentially, at suitable particle sizes, beta-glucans are easily phagocytosed by macrophages, which then release them, along with their metabolites, in much smaller particles that stimulate the production of anti-inflammatory cytokines, mainly interleukin-10, and proportionally a smaller production of inflammatory cytokines, mainly interleukin-6 and TNF-alpha. With this, a modulation of the immune system is obtained, resulting in the prevention or cure of chronic processes that are harmful to health.

Contrary to popular belief, inflammation is an essential process for maintaining homeostasis in humans, and its evolution to a state of chronic inflammation becomes harmful, often triggering or aggravating diseases. In diseases considered "the evils of the century", such as depression, anxiety, diabetes, obesity, gastrointestinal and cardiovascular diseases and even cancer, the installation of chronic inflammation, often subclinical, is one of the triggering factors.

The liver is the main organ responsible for the metabolism of food and xenobiotics, for the storage of minerals such as iron, and vitamins, such as vitamin A, for the production of enzymes responsible for digestion and for proteins that signal the individual's health status, in view of different inflammation conditions, such as C-Reactive Protein (CRP), along with the most diverse diseases, such as cancer, viruses, diabetes and obesity. Excess or deprivation of macroelements, such as glucose and fatty acids, and microelements, such as zinc, selenium and magnesium, contribute to the onset or worsening of diseases, which can become chronic due to a combination of two factors; oxidative stress and inflammation. However, the low hepatic activity may be related to the lesions caused by the aforementioned factors, in which the mycotoxins present in foods, especially grains, dairy products and vegetables, in addition to alcohol, favor this hypo or hyperfunction of the liver. Beta-glucans, in addition to being potent activators of the immune system, also act as broad-spectrum mycotoxin adsorbents. However, its efficiency is just over 50%, so that even with the continuous intake of beta-glucans, a part of the mycotoxins ends up reaching the liver, where their action is usually hepatotoxic, immunosuppressive and carcinogenic. Therefore, it is also necessary to use a liver protector such as silymarin, which has an anti-inflammatory and immunomodulatory action on hepatocytes, thus neutralizing the action of mycotoxins.

Oxidative stress is defined as the excess generation and/or deficiency in the removal of EROS (superoxide, hydroxyl ions, peroxides and hydroperoxides, for example) and ERNs (peroxynitrites) by intracellular antioxidant systems. The generation of EROS in the liver and other tissues is natural to cellular metabolism, such as the EROS generated in oxidative phosphorylation during glucose metabolism, and this entire process takes place in the mitochondria. Oxidative damage is a result of the high production of ROS by the mitochondrial respiratory chain, which leads this organelle to a dysfunctional state, contributing to the installation of many neurodegenerative (Alzheimer's) and metabolic (obesity, cancer and type 2 diabetes) diseases, resulting in the mitochondriopathies, which are morphological and functional alterations of mitochondria. The mitochondrial content of the cell is marked by the balance of biogenesis (mitochondrial fusion or fission) and mitochondrial degradation, which requires the regulation of the expression of nuclear (PGC1-α and NRF1) and mitochondrial (TFAM) genomes, in response to metabolism and the energy demand of the cell. Mitochondrial and nuclear transcription factors are the regulators of these processes. PGC-1alpha (*Co-activator-1'alpha' of the peroxisome proliferator-activated receptor*) is the main regulator of mitochondrial biogenesis, which in turn acts as a co-activator of NRF1 and NRF2 (Nuclear Respiratory Factor 1 and 2) that regulate the expression of electron transfer chain (ETC) subunits encoded by the nuclear genome, and bind to promoter genes such as TFAM (Mitochondrial Transcription Factor A) that drives transcription and replication of mitochondrial DNA (mtDNA).

In the last 50 years, the thesis that the genetic origin of cancers predominated. However, recently, support has grown for the thesis that cancer is actually a metabolic disease. Its origin would be in the loss of energy charge of the cells, when it drops below 56 kJ/mol. Below this level, a series of mutations begins in the genome, to adapt to the new energetic condition of the cells, creating cancer cells, which are those that manage to stop depending on oxidative phosphorylation, to depend on the anaerobic phosphorylation of substrates rich in glucose or glutamine. As in most cases, these cancer cells have dysfunctional mitochondria, the reduction of intracellular glucose, due to endocrine modulation provided by sleep, causes them to starve and to have a low multiplication rate, allowing greater effectiveness and success in chemotherapies.

Using natural products as pharmacological strategies capable of intervening in transcription factors and regulatory proteins related to the control of mitochondrial biogenesis can be a viable alternative, since mitochondriopathies are common to the most varied diseases, whether of immunological or metabolic origins.

The modification of the intestinal microbiota, aiming at a greater integrity of the intestinal epithelium, can be done through the daily intake of prebiotics specific to the genera of bacteria whose populations are desired to increase, which are lactobacilli and bifidobacteria. The ability of prebiotics to multiply populations of these genera of probiotic bacteria is known as a bifidogenic effect. They are present in many natural foods, but in insufficient amounts to significantly increase the populations of the desired bacteria. In addition, there are two problems in trying to increase the population of bacteria by just consuming foods rich in prebiotics:
- The need to vary the diet: in modern times, we are used to vary the diet almost daily, so that it would be impractical, or even impracticable, to maintain a strict diet over a period of years.
- Calorie Intake: Many of the foods, of which prebiotics are a part, are high in calories, so continuous intake of large amounts daily would exacerbate the problem of obesity.

The solution then is to consume an extra amount of these specific prebiotics, synthesized industrially, in order to obtain a significant increase in the desired bacteria, without incurring the risk of obesity and without interference from the diet.

There are two desirable genera of bacteria that act as commensals on the intestinal epithelium, protecting it against other bacteria in the intestinal microbiota. These are the *Lactobacillus* and *Bifidobacterium* genus. The prebiotics preferred by lactobacilli are FOS and inulin, as only bacteria of the genus *Bacillus* and some yeasts can ferment them. The prebiotics preferred by bifidobacteria are GOS and lactulose. Polydextrose and XOS also have a bifidogenic effect, but less than the former.

With increasing populations of lactobacilli and bifidobacteria, there is a competitive exclusion of populations of undesirable bacteria, such as *Clostridium, Salmonella* and *E. coli.* In addition, there is increased protection of the intestinal endothelium and increased production of short-chain fatty acids that serve as fuel for various cellular processes, such as glucose oxidation, rather than its conversion to fat in the liver. Furthermore, the size of populations of these beneficial bacteria determines the level of activity of the intestinal epithelial immune system.

GOS [galacto-oligosaccharides], lactulose, inulin and FOS [fructo-oligosaccharides] are the most studied prebiotics and are recognized as having the greatest bifidogenic effect, benefiting mainly bacteria of the genus *Bifidobacterium,* followed by *Lactobacillus,* allowing keep these populations high, thus avoiding the so-called "traveler's diarrhea", resulting from sudden changes in eating habits. With high populations of these two genera of bacteria, the protection and integrity of the intestinal epithelium is maintained, as well as the homeostasis of the immune system.

The bifidogenic effect on the intestinal microbiota is associated with the growth of bifidobacteria considered beneficial to health, thus managing to reduce the activity of putrefactive bacteria. *Bifidobacterium* is a genus of anaerobic bacteria that can act as probiotics. They represent one of the largest groups of bacteria that make up the intestinal microbiota and reside in the colon, promoting benefits for human health, as they are important in the prevention of cancer, gastrointestinal diseases or even allergies. Prior to the 1960s they were collectively known as *Lactobacillus bifidus.*

Glucans are linear, unbranched polysaccharides composed of beta-glucan units, joined by (1 → 3) and (1 → 4) bonds, whose molecular irregularity is reflected in their water solubility property. β-glucans comprise a group of β-D-glucose polysaccharides that can occur in the cell walls of grains and yeast. In grains, they are more concentrated in the sub-aleuroma layer, starchy endosperm and aleuroma layer. Beta-glucans are resistant to digestive processes, form viscous solutions on contact with water, and are pseudoplastic.

The 1,3 and 1,6 β-glucans are natural molecules capable of significantly improving our health. They represent highly conserved structures, often called pathogen-associated molecular patterns (PAMPs) or MAMPs. β-glucans are considered one of the main MAMPs for the detection of PRR (Pattern Recognition Receptor) mediated fungal infection. So far, the most important PRRs for β-glucans are the dectin-1 receptor, complement receptor 3 (CR3) and *Toll-like receptors* (TLR), found on various immune cells such as monocytes, macrophages, and dendritic cells, neutrophils, eosinophils and NK cells, but also in intestinal epithelial cells. Several cells in our body, in addition to the immune system, also recognize β-glucans and are influenced by them, such as fibroblasts. Alveolar epithelial and vascular endothelium cells also recognize these molecules.

The binding of β-glucans to dectin-1 induces a cascade of innate and adaptive immune responses, such as phagocytosis, production of ROS and production of cytokines and chemokines, by dendritic cells and macrophages, thus increasing resistance to infections.

Several studies report the potential health effects of β-glucans. Oat fiber, which contains β-glucans, when taken at least 3 g/day, can lower blood saturated fat levels and reduce the risk of heart disease, and act as immunomodulators.

β-glucans represent arrangements on the six sides of D-glucose rings connected linearly at each carbon position, varying by source, although more commonly β-glucans include 1,3-type glycosidic bonds in their structure. Although theoretically β-glucans are D-glucose polysaccharides linked by β -type glycosidic bonds, not all β-D-glucose polysaccharides are categorized as β-glucans. For example, cellulose is not a typical β-glucan as it is insoluble and does not have the same physicochemical properties as other cereal or oat β-glucans. Likewise, β-glucans from the cell wall of yeasts contain a much higher proportion of 1,3 and 1,6 β-glucans when compared to glucans from cereals. The 1,3 and 1,6 β-glucans from yeasts have different qualities, being more indicated as adsorbents of mycotoxins and modulators of the immune system. While cereal β-glucans, because they have a lower proportion of 1,3 and 1,6 β-glucans, are more suitable for the food and cosmetics industry.

For the industrial extraction of beta-glucans and mannans, which are part of the cell wall structure of yeasts and various fungi, the most common and desirable thing is that they are extracted from yeasts of the *Saccharomyces* and *Candida* genus. Depending on the method of preparation and their granulometry, 1,3 and 1,6 β-glucans may have more potential as adsorbents for mycotoxins, present in foods, or as an immunostimulant. Most mycotoxins have hepatotoxic, carcinogenic, nephrotoxic and teratogenic, and immunodepressive effects. Therefore, the use of beta-glucans is essential for any effort to modulate and activate the immune system.

When in microparticulate form, that is, when its particles have an average diameter greater than 100 micrometers, beta-glucans are more suitable for use as mycotoxin adsorbents, as they present greater difficulty in being phagocytosed and subsequently eliminated in even smaller particles. When beta-glucans have particles with an average diameter of less than 100 micrometers (µm), beta-glucans present themselves as activators of the immune system.

This is because when these small particles of beta-glucans reach the intestinal epithelium, as they contain protein fragments from yeast cell walls, they are perceived by APCs (Antigen Presenting Cells) cells. Macrophages are then mobilized to carry out their phagocytosis and are then released into the lymphatic system in even smaller particles. The microparticulate beta-glucans up to 100 µm can also permeate the mucus barrier and reach the intestinal epithelium, activating the immune system.

In addition, particulate 1,3 and 1,6 β-glucans promote T cell differentiation into Th1 cells and enhance initiation of cytotoxic T lymphocytes. The activation of Th1 lymphocytes is essential to maintain the body's wakefulness state, against invasions by microorganisms or various xenobiotics.

*Silybum marianum* (L.) Gaernt (Sm), commonly known as Silymarin, Milk Thistle or Milk Thistle is a plant in the Asteraceae family, native to the Mediterranean region and now growing and cultivated throughout the world. Its use was recorded by the Roman naturalist philosopher Pliny the Elder (23-79 A.C), who wrote that mixing the juice of this plant with honey was indicated to "bring bile". The medical use of silymarin was reported in the Middle Ages in Saxon records to ward off snakes and treat infectious diseases contracted after being bitten by a rabid animal. In the 16th century, two famous English herbalists, John Gerard and Nicholas Culpeper, recommended the use of Silymarin against all melancholic diseases and to cure fever, respectively.

Its leaves and seeds can be transformed into dry extract or standardized dry extract having as a marker some of its flavonolignans such as: silylbin (silibin), isosilibin A and B, silydianin, silicristin and 2,3-dihydro-silicristin (DHSB) in addition to of flavonoids.

The main pharmacological properties known of silymarin are its hepatoprotective function, followed by antidepressant. But pharmacological effects such as, anti-inflammatory, antidiabetic; antioxidant, antifibrotic, anticancer, cardioprotective and neuroprotective substances have also been studied in *in vivo* and *in vitro models.*

The anti-inflammatory effects of silymarin are due to the suppression of NF κ-B, including acyclooxygenase-2 (COX-2), prostaglandin E2 (PGE2) and production of inflammatory cytokines; and immunomodulatory by inhibiting the ERK pathway that acts in the recruitment of lymphocytes. The antidiabetic effect, by inhibiting Protein tyrosine phosphatase 1B (PTP1B), which negatively regulates the insulin and leptin signaling pathway. Studies show that with the inhibition of PTP1B, by the use of silymarin extract, there was an improvement in glucose tolerance and insulin sensitivity and in the inhibition of gluconeogenesis in the liver.

The antioxidant activity of silymarin has been shown to induce SOD (Superoxide Dismutase) activity and increase the content of Glutathione (GSH), which functions as a para-cellular homeostatic buffer.

The anti-fibrogenic activity of silymarin was demonstrated by the suppression of procollagen-α1 and TIMP-1, probably via negative regulation of TGF- β1mRNA, NF κ-B and by genes involved in the organization of the cytoskeleton of liver tissue, which shows its action against the fibrosis present in non-alcoholic steatohepatitis or NASH.

In a study performed with Swiss mice, intraperitoneal administration of silymarin extract at different doses (150, 200, 250 mg/kg; i.p.) showed an antidepressant effect (effective doses: 200, 250 mg/kg; i.p.), as it regulated circulating serotonin and dopamine levels. It also showed an antioxidant effect, by altering the levels of molecules involved in oxidative stress, such as MDA - Malondialdehyde and GSH-Glutathione. Other studies demonstrated that the use of a standardized dry extract of silymarin with silybinin for 5 days in BALB/c mice led to increased levels of dopamine and noradrenaline in the cerebellum. Another *in vitro study* demonstrated that siliinin inhibits the activity of monoamine oxidase (MAO), which catalyzes the oxidative deamination of monoamines such as dopamine and serotonin.

FOS and inulin belong to the group of oligosaccharides called fructans, and FOS is obtained by transfructosylation of sucrose, by the action of β-fructofuranosity (invertase) and fructosyltransferase enzymes, produced by many fungi, such as *Aspergillus niger, Aspergillus sp., Aureobasidium sp.* This process generates a mixture with the general formula GFn (1 glucose linked linearly to "n" fructoses), with n ranging from 1 to 5. All these isomers and oligomers are called FOS. Inulin is extracted from some foods where it occurs in large quantities, such as blue agave and chicory. Inulin has a general formula that brings together oligomers ranging from GF11 to GF60. *In vitro* propagation studies show that the higher the concentration of short-chain polymers in FOS, that is, the lower the n in the general formula GFn, the greater the bifidogenic effect. This means that FOS made up of short-chain polymer chains, such as GF2, GF3 and GF4, cause greater growth in the population of bacteria of the *Bifidobacterium* and *Lactobacillus* genus.

The genus *Lactobacillus* is divided into more than 50 species that colonize the entire intestinal epithelium, each inhabiting a pH range of the small and large intestines. While the genus *Bifidobacterium* is divided into approximately 30 species, most of which colonize segments of the large intestine. These two species act together, as the bifidobacteria produce proportionally more butyric acid, which is transformed into mucus by the goblet cells of the intestinal epithelium, and the lactobacilli produce proportionally more lactic and propionic acids, which are used to acidify the intestinal epithelium and as a source of energy by hepatocytes, thus repelling large intestine microbiota bacteria, which prefer neutral pH. In the segments of the small intestine, the defense strategy of lactobacillus is based on the use of bacteriocins against undesirable bacteria. Therefore, they act as "watchers and regulators" of the intestine in its integrity. When there is a break in the balance in this symbiotic partnership, through the use of antibiotics or foods rich in fat or carbohydrates, a dysbiosis occurs with the reduction of their populations and biochemical reactions begin, with the generation of oxygen species (ROS or ROS) and nitrogen. (ERN or RNS), which contribute to the initiation of the inflammatory process which, in turn, will result in a decrease in the protective mucus of the intestinal epithelium, making it susceptible to the action of pathogenic bacteria, such as those of the *Escherichia, Salmonella* and *Clostridium* genus. Which starts the whole cascade of neuro-immuno-endocrine system dysregulation in the gut. Installing morphological and histological changes, such as the decrease in absorption crypts (resulting in low absorption of nutrients, including minerals) and the decrease in colonization by the formation of fibrosis along the intestinal wall, which results in low colonization of these areas. As a result, there is a low activity of beneficial bacteria in the production of vitamins (such as vitamin K and B) and other essential metabolites for health.

FOS and inulin are present in many plants and foods such as bananas, onions, garlic, asparagus, honey, leeks, as well as grains and cereals such as wheat and barley. The highest concentrations of FOS are found in chicory, artichoke, yacon and blue agave. In Japan, since 1990, FOS is recognized as an important prebiotic, capable of increasing the health of the gastrointestinal tract, having been proposed as a supplement for the treatment of fungal and bacterial infections.

Many studies show that FOS and inulin promote increased absorption of calcium and other minerals such as magnesium in the small intestine. This is due to the fermentation of these saccharides by the intestinal microbiota, resulting in a lower or acidic pH. As the minerals are more soluble in acidic pH, they allow a better use by the body, thus being able to be better absorbed to enter the bloodstream. In addition, magnesium is especially important in the process of serotonin production by enterochromaffin cells, in addition to being an important regulator of mood, by increasing the absorption of magnesium and calcium, by regulating glycolytic metabolism, as it directly participates in biochemical reactions involving production and secretion of neurotransmitters.

Galacto-oligosaccharides (GOS) are branched chains of oligosaccharides formed with galactose and glucose molecules. They are found in the first food offered to babies, in breast milk. The prebiotics GOS and lactulose are known as oligogalactosylactose, oligogalactose, lactulose, oligolactose or transgalactooligosaccharides (TOS) and belong to the class of bifidogenic prebiotics, as they favor the growth of bifidobacteria and lactobacilli. The composition of the galactooligosaccharides (GOS) fractions varies according to the chain length and type of bonds between the monomeric units. In general, they are produced through the enzymatic conversion of lactose, a component of bovine milk, by the enzyme β-galactosidase. But they can also be produced by chemical isomerization of lactose, as is the case with lactulose or be naturally present in soy.

Polydextrose is an oligosaccharide composed only of glucose molecules. XOS (xylo-oligosaccharide) is produced from xylose, which is a saccharide extracted from plants rich in hemicellulose. Both have a moderate bifidogenic effect and tend to be more competitively priced than FOS and GOS.

Document BR20161014961 teaches formulation for veterinary use comprising oligosaccharides such as FOS, GOS, MOS, 1,3 and 1,6 beta-glucans. The formulation was developed to replace antibiotic growth regulators in animal husbandry.

Document CN109452370 teaches milk powder formulation for students comprising milk powder, whey protein, concentrated powder, whey, vegetable oil, maltodextrin, sugar, phospholipids, vitamins, minerals, lactalbumin, preservative, FOS and bifidobacteria. Particularly, it employs nutrients like active probiotics, prebiotics like GOS, FOS, unsaturated fatty acids, vitamin A, D, iron, zinc, calcium and taurine.

Document WO2019153334 teaches a prebiotic composition comprising inulin, GOS, xylitol and wheat beta-glucan, for enhancing intestinal immunity. The proportion between the components is different from that used in the present invention, in addition to not comprising silymarin. The composite prebiotic composition employed may contain 70-150 parts of inulin, 20-100 parts of galactooligosaccharides, 1-50 parts of xylitol, 0,05-3 parts of yeast β-glucan and 5-40 parts of xylooligosaccharides. In another specific embodiment of the invention, based on parts by weight, the composite prebiotic is composed of 75 parts of inulin, 23 parts of galactooligosaccharides, 2 parts of xylitol, 0,1 parts of yeast β-glucan and 8 parts of xylooligosaccharides.

Document CN 107232610 teaches composition of sugars containing beta-glucan and oligosaccharides which can be isomalto-oligosaccharide, lactose-oligosaccharide, GOS and XOS, in addition to comprising other polymers of monosaccharide, disaccharide, polysaccharide, maltodextrin and glucose. The mixture has application in foods with use in special medicines.

Document US6241983 teaches composition for promoting gastrointestinal health comprising microorganisms and dietary fiber. Microorganisms are present in the composition at 0,1 to 20% by weight; dietary fiber by 40 to 60% by weight; and further 40 to 60% by weight of immunoglobulin, preferably of bovine origin. Dietary fiber is a selected member of the group consisting of pentosans, beta-glucans, pectins and pectic polysaccharides, mannans, arabinans and galactans, fructooligosaccharides and mixtures thereof. Preferred beneficial human intestinal microorganisms include lactobacilli and bifidobacteria.

Document CN107927788 deals with oligosaccharide nutrient prepared from raw materials as follows: glucan syrup, polydextrose, fructooligosaccharide, xylooligosaccharide, galactooligosaccharide, isomaltooligosaccharide, lactulose, vitamin B6, vitamin C, magnesium sulfate, calcium lactate and salt of ferric sodium from EDTA. The formulation improves the health of the intestinal tract, as well as bringing benefits to the skin.

Document US 8318218 B2 deals with the use of 1,3 and 1,6 beta glucans as a slimming or weight loss agent in the treatment of obesity. Beta-glucans from *Saccharomyces cerevisae* may be associated with numerous plants or herbs, including Silymarin *(Silybum marianum).* The document also mentions the possibility of using other probiotics, such as FOS, but does not indicate levels. According to this document *Silybum ebumeum* or *Silybum marianum* relieves symptoms of constipation by improving bowel function, and constipation is often associated with individuals who are obese. In particular this herb improves digestive levels and energy levels. Paragraph [0045] of the original document comments that the amount of herb can vary from 25 to 100 mg per dose of the composition. In paragraph [0046] of the same document the ratio between herbs (in total) and glucans (in total) is indicated, which preferably varies from 1:1 to 10:1, more preferably 1,5:1 to 5:1 and especially 2:1 to 4:1.

### Brief description of the figures

Below is a brief description of the attached figures:
Figure 1 depicts a panel demonstrating the hen embryonated egg chorio-allantoic membrane assay (HET-CAM) with Saline Solution (NaCl) as a negative control, 1M Sodium Hydroxide (NaOH) as a positive control and the Icarian Composition, in triplicate of use example 2 of this invention.
Figure 2 shows liver tissue slides showing the levels of steatosis, according to the use of the composition in the guinea pig feed, from use example 2.
Figure 3 shows a microphotograph of liver tissue with different levels of fibrosis, according to the use or not of the composition in the guinea pig ration, in use example 2.
Figure 4 shows cross-sections of the intestinal epithelium of guinea pigs, where the effect of the use of the composition in the feed on the morphology of goblet cells can be observed, in example of use 2.

### Synthesis of the invention

A composition of beta glucans and a phytotherapic and prebiotics was developed, with neuro-immune-endocrine regulator, antioxidant and growth promoter of beneficial bacteria of the intestinal microbiota, with significant effects of improving sleep quality, but with different mechanisms of action, comprising the following components indicated below:
- 1,3 and 1,6 beta-glucans;
- Silymarin (*Silybum marianum*)*,* or its natural or synthetic flavonolignans or flavonoids;
- one or more prebiotics with a bifidogenic effect.

### Objectives of the Invention

A composition was developed to be used as a nutraceutical supplement, which had the following properties or advantages:
- comprised in its formulation specific amounts of beta-glucans and a phytotherapic and one or more bifidogenic prebiotics, with distinct but synergistic modes of action, in order to be able to act on several fronts in the broad spectrum of bacteria of the intestinal microbiota, aiming to increase the protection and integrity of intestinal and liver epithelium;
- represented a modulating product of the neuro-immune-endocrine axis, acting on three simultaneous fronts:
   * improving emotional balance, by regulating the release of adequate amounts of neurotransmitters involved in the sleep-wake cycle and thus providing greater quantity and quality of sleep;
   * modulating and activating the immune system, with increased production of anti-inflammatory cytokines (IL-10) and reduced production of inflammatory cytokines (IL-6) and TNF-alpha. Thus reducing chronic inflammation, which impairs sleep; and
   * regulating and protecting the endocrine system, with activation of mitochondrial biogenesis, in order to prevent or eliminate oxidative stress that involves the entire body, especially the liver and, with this, contributing to the improvement of the metabolic functions of carbohydrates and lipids, the sleep and the functioning of the immune system.

### Description of the invention

The neuro-immuno-endocrine modulating composition of this invention is intended to significantly improve sleep quality by increasing the proportion of NREM deep sleep (SWS), especially NREM stage 3 and 4. Each component of the composition has a distinct mechanism of action and there is a synergy between these mechanisms of action. The composition comprises the essential components listed below, each of which represents one of the mechanisms of action:
- 1,3 and 1,6 beta-glucans;
- Silymarin (*Silybum marianum),* or its natural or synthetic flavonolignans or flavonoids; and
- one or more prebiotics with a bifidogenic effect.

The 1,3 and 1,6 beta-glucans, which are part of this invention, are intended to modulate and activate the immune system, especially the innate immune system, in addition to adsorbing part of the mycotoxins present in foods, mainly grains, dairy products and vegetables, in a to reduce liver damage. The maximum effect is obtained when using 1,3 and 1,6 beta-glucans that show anti-inflammatory action in small doses, that is, that increase the production of IL-10 in a greater proportion than the inflammatory cytokines IL-6 and TNF-alpha.

Silymarin, which composes this invention, is intended to act as a liver protector, protecting hepatocytes from the hepatotoxic and immunosuppressive action of toxins, in addition to acting as an antidepressant, due to its proven effects in raising noradrenaline and dopamine levels and in inhibiting deamination. oxidation of serotonin and dopamine in the brain. Silymarin can be used through leaves and seeds that are transformed into dry extract or standardized dry extract, comprising flavonoids and flavonolignans. Some of the silymarin flavonolignans that can be used in natural or synthetic form are indicated, namely: silymarin, silybin A, silybin B, isosilibin A, isosilibin B, silycristin A, silycristin B, isosilicristin A, dehydrosylristin A, silydianin, dehydrosilidianin, and 2,3-dihydro-silicristine (DHSB) in addition to flavonoids.

For the purpose of this invention, prebiotics with a bifidogenic effect have the same mechanism of action, that is, to increase the populations of lactobacilli and bifidobacteria that inhabit the entire intestinal tract. The maximum effect of the composition is obtained when using several associated prebiotics, preferably FOS and GOS in defined proportions. To a lesser extent, the composition may also contain other prebiotic components such as: polydextrose, lactulose, inulin, XOS (xylo-oligosaccharides), and/or MOS (mannono-oligosaccharides) among other prebiotics with a bifidogenic effect.

The prebiotics with bifidogenic effect, which make up the invention, are intended to promote a marked growth in the populations of more than 50 species of lactobacilli, and more than 30 species of bifidobacteria, existing throughout the TGI, especially in the cecum. With this, it is intended to carry out the competitive exclusion of undesirable bacteria, which can adhere to the intestinal epithelium, resulting in dysbiosis or more serious inflammatory bowel diseases, contributing to the neurobiology of insomnia, modulating the serum level of cortisol and controlling weight gain. In addition, the bifidogenic prebiotics of the composition of this invention have the purpose of modulating the endocrine system and the metabolism of carbohydrates and lipids in the fatty liver, as well as increasing the populations of the species of bacteria that make up the psychobiotic microbiota, in order to promote the increase the production of dopamine and serotonin in the brain.

According to one embodiment of the invention, the immune system modulating and activating composition of this invention comprises the following essential components:
- 1,3 and 1,6 Beta-glucans from the cell wall of yeasts of the *Saccharomyces* and/or *Candida genera;*
- Silymarin *(Silybum marianum),* or its natural or synthetic flavonolignans or flavonoids.
- one or more prebiotics with a bifidogenic effect chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and /or Mannan-oligosaccharide (MOS) and/or glyco-oligosaccharides, their subspecies or subtypes, and/or other prebiotics with bifidogenic action.

According to another embodiment of the invention, the immune system and lipid and carbohydrate metabolism (immunometabolism) modulating composition of this invention comprises the following components:
- 1,3 and 1,6 beta-glucans;
- Silymarin (*Silybum marianum*)*,* or its natural or synthetic flavonolignans or flavonoids;
- one or more prebiotics with a bifidogenic effect;
- Zinc and/or magnesium and/or selenium minerals.

The central thesis of this invention is that there is a synergy between the effects of the components of the composition, when used together and in the correct proportions. In addition, the joint action of these components is sufficient to keep the immune system modulated and active, in order to reduce the effects of pre-existing inflammation, improve metabolic and physiological indicators, reduce the body's oxidative processes and protect the integrity of the epithelium. intestine and liver. Finally, it is sufficient to promote a significant improvement in the factors that determine a good quality of sleep, with an increase in the proportion of NREM 3 and 4 deep sleep.

Many medications purport to fight insomnia, such as melatonin, hypnotics and anxiolytics. However, according to experts, none provide lasting improvement in sleep quality and, mainly, an increase in the amount of deep sleep. Only immuno-neuroendocrine regulation, which restores homeostasis, can provide a significant and lasting improvement in sleep, in terms of quality and quantity.

Other expected effects from using the composition of this invention are:
*function as a regulator of intestinal serotonin production, in order to improve peristalsis;
* promote increased production of growth hormone, to assist in cell renewal;
* modulate mitochondrial biogenesis, reducing the amount of intracellular glucose;
* protect the integrity of the enteric nervous system and autonomic nervous system;
* modulate circulating lipoproteins LDL, HDL, in addition to cholesterol and triglycerides;
* act as an antioxidant, protecting cell organelles against ROS, ERNs and molecules capable of causing inflammatory processes, which contribute to premature aging.

Below are presented 3 different application examples already carried out with the isolated or associated components of the composition of this invention. Example 1 shows that, in isolation, all components of the composition showed effects on oxidative stress and inflammatory and anti-inflammatory cytokines. However, the comparison of their individual performances with that of the composition of this invention, makes clear the superiority of the latter. Example 2 shows the neuroendocrine regulatory effects of the composition of this invention on an experimental model with obese and insulin resistant C57B6 mice. Finally, Example 3 shows the effects on sleep quality and modulation of the endocrine system observed in volunteer patients who consumed capsules of the composition of this invention.

The composition of this invention can be consumed in the form of capsules, tablets or sachets.

Complementing the neuro-immuno-endocrine modulating and regulating function of the population of amphibiotic bacteria of the intestinal microbiota and protecting the liver, the composition of this invention may contain mineral micronutrients, in inorganic forms or associated with organic molecules, preferably chelated zinc and magnesium and organic selenium, whose functions are vital for the metabolic functioning of the human being.

The 1,3 and 1,6 beta-glucans of the composition of this invention are responsible for modulating the immune system, in addition to acting as mycotoxin adsorbents, and must come from the extraction of the cell wall of yeasts of the genera *Saccharomyces* or *Candida,* preferably from *Saccharomyces cerevisiae* from the production of ethanol from sugar cane. It has now been discovered that the particle size of beta-glucans is very important to achieve a high degree of absorption or good bioavailability in relation to the microvilli of the absorptive cells or enterocytes of the intestine. For this reason, the 1,3 and 1,6 beta-glucans according to the invention must be industrially microparticulated, with the aim of having an average particle diameter of less than 100 microns.

Example 1 below indicates two sources of beta-glucans from *Saccharomyces cerevisae* that were tested in isolation. One is commercially called YES GlucanMOS, whose 1,3 and 1,6 beta-glucans (hereinafter called beta-glucans) have an average particle size of 300 microns, and the other type of beta-glucans is commercially called YES GlucanGold and has an average particle size of 60 microns. GlucanGold is the beta-glucan that represents the composition, according to the invention in the example. YES GlucanMOS did not participate in the composition of this invention, having been exemplified only for isolated use purposes. As can be seen, YES GlucanMOS, FOS and GOS have little or no effect on their own. YES GlucanGOLD has a good effect. However, the composition has much more effect or produces results, when compared at the same dosage, due to the synergistic effect. That is, 257 mg of each component, alone, has much less effect than 257 mg of the composition, and in 257 mg of the composition there are approximately 90 mg of FOS, 90 mg of GOS and 67 mg of YES GlucanGOLD.

According to a preferred embodiment of the invention, the regulatory composition of the neuro-immune-endocrine system, promoting the improvement of sleep quality, comprises the following components:
- 1,3 and 1,6 beta-glucans, from the cell wall of yeasts of the *Saccharomyces* and/or *Candida genera,* with particles with an average diameter of less than 100 microns;
- Silymarin (*Silybum marianum*)*,* or its natural or synthetic flavonolignans or flavonoids;
- all polymers of generic names of prebiotics with bifidogenic effect. For example, in GOS (galactooligosaccharides), all polymers of this denomination that have a bifidogenic effect are included, including lactulose. In FOS (fructooligosaccharides), all polymers of this denomination that present a bifidogenic effect are included, including inulin. And Polydextrose includes all glycooligosaccharides. In summary, one or more prebiotics with a bifidogenic effect chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and/or Mannan-oligosaccharide (MOS) and/or glyco-oligosaccharides, their subspecies or subtypes, and/or other prebiotics with bifidogenic action.

In an even more preferred way, the regulatory composition of the neuro-immune-endocrine system, promoting the improvement of sleep quality, comprises the following components:
- 1,3 and 1,6 beta-glucans, from the cell wall of yeasts of the *Saccharomyces* and/or *Candida genera,* with an average particle diameter of less than 100 microns;
- Silymarin (*Silybum marianum*)*,* or its natural or synthetic flavonolignans or flavonoids;
- all polymers of generic names of prebiotics with bifidogenic effect. For example, in GOS (galactooligosaccharides), all polymers of this denomination that have a bifidogenic effect are included, including lactulose. In FOS (fructooligosaccharides), all polymers of this denomination that present a bifidogenic effect are included, including inulin. And Polydextrose includes all glycooligosaccharides. In summary: one or more prebiotics with a bifidogenic effect chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and/or Mannan-oligosaccharide (MOS) and/or glyco-oligosaccharides, their subspecies or subtypes, and/or other prebiotics with bifidogenic action.
- minerals: Zinc and/or Magnesium and/or Copper and/or Selenium, in their inorganic forms or associated with organic molecules.

The silymarin of this invention is responsible for protecting the integrity of the liver against damage caused by hepatotoxic mycotoxins, preserving the proper functioning of the organ, in addition to having an antidepressant effect, protecting against the deamination of dopamine and serotonin. For the purposes of this invention, any extract or powder of the plant *Silybum marianum* is considered usable for the composition. Silymarin leaves and seeds can be made into a dry powder for use comprising flavonoids and flavonolignans.

The percentages of mass in relation to the total mass of the composition, among the pure components, are indicated in Table 1 below:

**TABLE 1**

| | 1,3 and 1,6 beta-glucans | Bifidogenic prebiotics | Silymarin |
|---|---|---|---|
| Maximum content | 2% | 38% | 4% |
| Minimal content | 10% | 70% | 11% |

According to a preferred embodiment of the invention, the contents of the pure components are indicated in Table 2 below:

**TABLE 2**

| | 1,3 and 1,6 beta-glucans | Bifidogenic prebiotics | Silymarin |
|---|---|---|---|
| Maximum content | 3% | 43% | 6% |
| Minimal content | 8% | 63% | 10% |

There is a range of ingestion dosages of the composition of this invention where results are considered optimal and safe. Table 3, below, shows the minimum and maximum limits of this range. The intake of the composition of this invention should be proportional to the live weight of the patients. Below the minimum threshold the effects are very small. Above the maximum limit increases the incidence of undesirable side effects such as SIBO (*Small Intestine Bacterial Overgrowth*) or disproportionate immune system reactions.

**TABLE 3**

| | Composition Intake Limits | |
|---|---|---|
| | (mg/kg BW/day) | per 70 kgPV/day |
| Minimum | 10,6 milligrams | 0,7 grams |
| Maximum | 44,3 milligrams | 3,1 grams |

According to another embodiment of the invention, the mass ratios between the components, pure, 1,3 and 1,6 Betaglucans:Bifidogenic Prebiotics:Silymarin can vary from 0,7 to 1,3 of 1,3 and 1,6 Betaglucans ; from 6,5 to 12,0 of Bifidogenic Prebiotics 0,8 to 1,4 of Silymarin. Preferably, the mass ratio between the components of the mixture is: from 0,9 to 1,1 of 1,3 and 1,6 Beta-Glucans; from 7,5 to 10,5 of Bifidogenic Prebiotics and from 0,9 to 1,3 of Silymarin. Even more preferably, the mass ratios between 1,3 and 1,6 Beta-Glucans:Bifidogenic Prebiotics:Silymarin obey the ratio 1:8,9:1,1 when expressed in whole numbers.

The proportions of 1,3 and 1,6 beta-glucans: Bifidogenic Prebiotics: Silymarin can change in the composition, object of this invention, according to the type of region or health problem that is intended to be solved. They can also change depending on how the composition is used, whether preventive or curative.

Among the various health improvements, which lead to improved sleep quality, that the composition of this invention can promote, we can mention:
- modulation of the immune system;
- modulation of the endocrine system;
- reduction of anxiety and depression states, by modulating the release of adequate amounts of serotonin.
- regulation of intestinal transit, acting in situations such as constipation or diarrhea;
- preservation of the integrity of the intestinal epithelium and liver;
- elevation of HDL cholesterol;
- reduction of triglycerides and total cholesterol;
- increase in lean mass and decrease in fat mass;
- reduction of visceral fat;
- flattening and shortening of the glycemic curve (modulation of glycemia and insulin);
- greater absorption of minerals.

Among the various situations in which the use of the composition of this invention can be recommended, we can mention:
- need to improve emotional well-being;
- need to improve sleep quality;
- need to increase the proportion of NREM 3 and 4 deep sleep;
- need to increase longevity;
- need for antioxidant protection;
- need for immunoregulation for patients undergoing chemotherapy;
- need to reduce glucose availability for cancer cells;
- need to control inflammation of the intestinal epithelium;
- need to assist in the treatment of diabetes;
- need to gain lean mass;
- need to assist in the treatment of hepatic steatosis;
- need to assist in the treatment of dyslipidemias.

The composition according to the invention acts in a prophylactic way on human health in general, contributing to improve the neuro-immune-endocrine system and protect the liver and intestinal epithelium, and thus helps in the treatment of various pathologies such as: tuberculosis, dengue, viruses.

It is particularly used as an aid to boost immunity in chemotherapy (cancer) treatments, and as an aid in the treatment of obesity and diabetes.

In diabetics, the consequences of infections with viruses such as Covid 19 and *Influenza* A are usually much more devastating. The increase in intracellular glucose results in the installation of oxidative stress and the final metabolism of fructose-6-phosphate to uridine diphosphate-N-acetyl glucosamine (UDPGIcNAc), and activation of the hexosamine pathway, resulting in pathological changes in gene expression, increasing the production of inflammatory cytokines and transcription factors, among which Interleukin 6 (IL-6) and TNF-alpha stand out. The excess of inflammatory cytokines favors the environment for the damage caused by the viral infection. The composition of this invention promotes a triple protective and regulatory action of the body, with beta-glucans adsorbing mycotoxins from food and preventing liver damage, and modulating the immune system; with silymarin having antidiabetic, anti-fibrogenic and immunostimulant action; with bifidogenic prebiotics promoting increased nutritional support for the proper functioning of the body, causing a greater amount of deep sleep that is due to the modulation of mitochondrial biogenesis that was activated by the regulation of transcription factors and by the anti-inflammatory action of said composition. There is greater hepatic activity, greater transformation of glucose into glycogen, greater glucose tolerance and greater insulin sensitivity in the body, which limits the activity of the hexosamine pathway and oxidative stress and, therefore, the excessive production of inflammatory cytokines. In addition, the microparticulate 1,3 and 1,6 beta-glucans of the composition lead to increased production of interleukin 10 (IL-10), which is anti-inflammatory. Thus, there is a modulation of the immune system, which is able to face viral and bacteriological challenges much more efficiently.

The treatment method employing the neuro-immune-endocrine system regulatory composition, according to the invention, has daily intake limits between 10,6 and 44,3 mg per kilogram of live weight, that is, between 0,7 and 3,1 grams per day per person of 70 kg bodyweight.

The regulatory composition of the neuro-immune-endocrine system, according to the present invention, can be used as an aid for the enhancement of immunity and for the restriction of glucose to tumors, in chemotherapy treatments, or as an aid in the treatment of obesity and diabetes, or be used in public health prophylaxis.

The examples below are merely illustrative of the invention and should not be taken for the purposes of limiting it. The name "Icaria" was used for each composition according to the invention represented in figures or tables.

### Example 1

### Objective: Demonstrate the Synergy between the Components of the Composition of this Invention

### Phase 1

In Phase 1, the experiment consisted of providing the same amount (in mg), per kg of live weight (mg/kg BW), of each of the components of the composition of this invention, as well as the same amount of the composition, and comparing the effects with that of control animals, which received placebo or the antibiotic Enramycin. After 10 days, an evaluation of the immune system of the mice of each of the groups was carried out.

The calculations of the dosage to be consumed by the rats were extrapolated from a daily intake, by humans with 70 kg of body weight, of 1,7 grams of the composition of this invention, in pure components (1,3 and 1,6 beta-glucans, bifidogenic prebiotics GOS and FOS, with 100% purity). Thus, for the purposes of this invention, human consumption, from which the calculations were made, is within the range considered ideal and safe to be ingested.

The total mass of the composition of this invention ingested by the rats was 257mg/kgBW. As it was composed of YES GlucanGold (containing 60% of 1,3 and 1,6 beta-glucans, microparticulates less than 100 microns in average diameter per particle), YES GOS (containing 70% pure GOS) and YES FOS (88% of pure FOS), the participation of the components of the composition, in mass over total mass %, was 20% of 1,3 and 1,6 beta-glucans, 23,3% of GOS and 31,3% of FOS. Therefore, within the limits set forth in this invention.

In this way, different groups of male *Wistar rats* were randomly marked on the tails for identification. The animals were adapted to the laboratory environment for one week before the treatments. Initially, after a 6 h fasting period, the body weight of the animals was determined and the dose of the Composition and Enramycin prebiotics were calculated. The animals were divided into 11 (eleven) groups of 10 (ten) individuals. The animals were treated daily, orally (used in the animals used as reference), with placebo (vehicle; filtered water 1 mL/kg BW) or with FOS Ingredion (257 mg/kg BW), FOS Yes (257 mg/kg BW) kgPV), GOS Yes (257 mg/kgPV), Yes MOS (257 mg/kgPV), Yes GlucanMOS (257 mg/kgPV), Glucangold Yes (77 mg/kgPV), Mixture 1 (FOS Yes + GOS Yes) (257 mg/kgPV), Composition of this invention (GlucanGold Yes + GOS Yes + FOS Yes, containing 20% of 1,3 and 1,6 beta-glucans, 29% of GOS and 31% of FOS by mass on total mass %) (257 mg /kgPV) or with Enramycin (0,96 mg/kgPV). An experimental group called naive underwent simulated gavage (without any treatment). All the animals that composed the different experimental groups received the treatment for 10 days. At the end of the experiments, and before euthanasia, the animals were fasted for 6 hours with free access to water. Final body weight was recorded to calculate relative organ weight. Then, under anesthesia with isoflurane (2-3%), 5 mL of blood was obtained from the left jugular vein for the evaluation of biochemical parameters. After this procedure, the animals were euthanized with a supra-pharmacological dose of isoflurane (10-15%) in a saturation chamber. Immediately after euthanasia, target organs were removed to determine relative weights and to assess tissue redox status.

Table 4 shows the effects of treatments of different components alone, with the Composition according to the invention, or with Enramycin, on the serum levels of redox state markers (niterorosin, nitrite and malondialdehyde) in male Wistar rats. The results shown in Table 4 show that the high serum levels of Nitrite and the low serum levels of Malondialdehyde of the group treated with the Composition of this invention were significantly better than that of all other groups/treatments. This demonstrates the strong antioxidant and anti-inflammatory action of the composition of this invention on the body's oxidative processes.

**TABLE 4**

| Experimental groups | Diary dose | Parameters | | |
|---|---|---|---|---|
| | | **Nitrite** (%) | **Nitrotirosine** (µmol/L) | **Malondialdehyde** (mmol/L) |
| Naive | 0 | 48,1 ± 4,22 | 0,011 ± 0,002 | 2,2 ± 0,10 |
| Placebo | 1 ml/kgPV | 50,2 ± 5,31 | 0,009 ± 0,003 | 2,2 ± 0,12 |
| FOS ingredion | 257 ml/kgPV | 45,9 ± 6,21 | 0,008 ± 0,002 | 2,0 ± 0,08 |
| FOS YES | 257 ml/kgPV | 69,8 ± 3,44^{abcd} | 0,010 ± 0,003 | 1,6 ± 0,08^{abcd} |
| GOS YES | 257 ml/kgPV | 61,5 ± 6,21 | 0,011 ± 0,002 | 1,7 ± 0,09^{abcd} |
| YES MOS | 257 ml/kgPV | 59,4 ± 4,87 | 0,009 ± 0,002 | 1,9 ± 0,11 |
| Glucan MOS YES | 257 ml/kgPV | 61,3 ± 5,99 | 0,008 ± 0,002 | 1,9 ± 0,11 |
| Glucan Gold YES | 257 ml/kgPV | 71,6 ± 4,33^{abcd} | 0,010 ± 0,002 | 1,7 ± 0,08^{abcd} |
| FOS+GOS YES | 257 ml/kgPV | 79,1 ± 3,11^{abcd} | 0,009 ± 0,003 | 1,5 ± 0,07^{abcd} |
| **Composition (*)** | **257 ml/kgPV** | **88,2 ± 3,27^{abcd}** | **0,012 ± 0,003** | **1,6 ± 0,08^{abcd}** |
| Enramicine | 96 ml/kgPV | 45,3 ± 5,51 | 0,007 ± 0,003 | 2,1 ± 0,15 |
| (*) Composition, according to the invention, comprising 20% of 1,3 and 1,6 beta-glucans, 29% of GOS and 31% of FOS, mass over mass % | | | | |
| The values are expressed as the average + standard error of the average. ANOVA of a second way followed by the Bonferroni test. **^{a}p < 0,05 in comparison to naive group; ^{b}p < 0,05 in comparison to placebo group; ^{c}p < 0,05 in comparison to FOS ingredion; ^{d}p < 0,05 in comparison to ^{a}p < 0,05 in comparison to naïve group.** | | | | |

The results shown in Tables 5 and 6, below, also show that the antioxidant action of the composition of this invention, in the Cecum and Colon, was significantly superior to that of the other groups/treatments.

**TABLE 5**

| Experimental groups | Diary dose | **LPO (U/mg of protein)** | | | |
|---|---|---|---|---|---|
| | | **Jejune** | **Ileum** | **Cecum** | **Colon** |
| Naive | 0 | 100,2 ± 9,2 | 124,2 ± 11,2 | 167,2 ± 10,5 | 212,2 ± 16,5 |
| Placebo | 1 ml/kgPV | 94,9 ± 10,2 | 110,2 ± 13,9 | 172,1 ± 11,2 | 199,2 ± 15,5 |
| FOS ingredion | 257 ml/kgPV | 107,4 ± 11,4 | 118,4 ± 12,2 | 180,2 ± 10,9 | 182,2 ± 9,2 |
| FOS YES | 257 ml/kgPV | 89,9 ± 11,5 | 127,5 ± 15,5 | 157,1 ± 10,2 | 147,2 ± 10,1^{abcd} |
| GOS YES | 257 ml/kgPV | 108,1 ± 10,8 | 120,2 ± 12,2 | 152,2 ± 13,2 | 157,2 ± 11,2^{abcd} |
| YES MOS | 257 ml/kgPV | 98,5± 12,5 | 118,2 ± 12,2 | 180,2 ± 15,2 | 169,6 ± 12,2 |
| Glucan MOS YES | 257 ml/kgPV | 100,2 ± 9,2 | 130,2 ± 10,2 | 161,8 ± 10,2 | 171,9 ± 16,2 |
| Glucan Gold YES | 257 ml/kgPV | 105,5 ± 10,5 | 119,9 ± 19,9 | 120,1 ± 9,0^{abcd} | 135,5 ± 11,1^{abcd} |
| FOS+GOS YES | 257 ml/kgPV | 99,9 ± 10,7 | 121,2 ± 11,9 | 117,2 ± 8,2 | 141,1 ± 12,1^{abcd} |
| **Composition (*)** | **257 ml/kgPV** | **112,2 ± 13,2** | **127,8 ± 17,5** | **121,22 ± 6,2^{abcd}** | **150,2 ± 10,3^{abcd}** |
| Enramicine | 96 ml/kgPV | 45,3 ± 5,51 | 117,9 ± 14,2 | 170,2 ± 10,0 | 230,2 ± 11,2 |
| (*) Composition, according to the invention, comprising 20% of 1,3 and 1,6 beta-glucans, 29% of GOS and 31% of FOS, mass over mass % | | | | | |
| The values are expressed as the average + standard error of the average. ANOVA of a second way followed by the Bonferroni test. **^{a}p < 0,05 in comparison to naïve group; ^{b}p < 0,05 in comparison to placebo group; ^{c}p < 0,05 in comparison to FOS ingredion; ^{d}p < 0,05 in comparison to ^{a}p < 0,05 in comparison to naïve group.** | | | | | |

Table 5 and Table 6 show the effects of treatments with different components alone, with the Composition, or with Enramycin, on tissue levels of lipoperoxides (LPO) in male Wistar rats.

**TABLE 6**

| Experimental groups | Diary dose | **LPO (U/mg of protein)** | | |
|---|---|---|---|---|
| | | **Liver** | **Spleen** | **Kidneys** |
| Naive | 0 | 151,1 ± 10,1 | 124,1 ± 10,1 | 108,4 ± 7,5 |
| Placebo | 1 ml/kgPV | 165,4 ± 12,1 | 115,5 ± 9,2 | 115,2 ± 8,1 |
| FOS ingredion | 257 mg/kgPV | 159,2 ± 11,4 | 112,5 ± 10,2 | 119,7 ± 9,7 |
| FOS YES | 257 mg/kgPV | 167,2 ± 13,2 | 119,8 ± 8,2 | 99,8 ± 8,1 |
| GOS YES | 257 mg/kgPV | 152,2 ± 11,2 | 120,2 ± 9,9 | 104,4 ± 7,7 |
| YES MOS | 257 mg/kgPV | 168,5 ± 13,3 | 109,5 ± 10,5 | 115,8 ± 8,0 |
| Glucan MOS YES | 257 mg/kgPV | 159,5 ± 11,1 | 118,9 ± 8,7 | 100,5 ± 8,8 |
| Glucan Gold YES | 257 mg/kgPV | 168,6 ± 10,9 | 123,5 ± 11,5 | 98,8 ± 9,0 |
| FOS+GOS YES | 257 mg/kgPV | 147,1 ± 12,2 | 115,8 ± 9,2 | 89,1 ± 7,6 |
| **Composition (*)** | **257 mg/kgPV** | **144,1 ± 13,5** | **121,8 ± 8,7** | **90,2 ± 8,1** |
| Enramicine | 96 mg/kgPV | 170,1 ± 14,1 | 117,5 ± 10,1 | 119,4 ± 7,1 |
| (*) Composition, according to the invention, comprising 20% of 1,3 and 1,6 beta-glucans, 29% of GOS and 31% of FOS, mass over mass % | | | | |
| The values are expressed as the average + standard error of the average. ANOVA of a second way followed by the Bonferroni test. **^{a}p < 0,05 in comparison to naive group; ^{b}p < 0,05 in comparison to placebo group; ^{c}p < 0,05 in comparison to FOS ingredion; ^{d}p < 0,05 in comparison to ^{a}p < 0,05 in comparison to Enramicine group.** | | | | |

The results shown in Table 7 prove that, with high statistical significance, the composition of this invention provided the highest production of interleukin 10 (IL10), which is anti-inflammatory, among all groups/treatments.

Likewise, with high statistical significance, there was a lower relative production of interleukin 6 (IL6) and tumor necrosis factor alpha (TNF-alpha), which are inflammatory cytokines. That is, the results of Table 7, below, show that the composition of the invention represents the treatment that had the greatest anti-inflammatory effect, that is, it was the one that most modulated the immune system.

**TABLE 7**

| Experimental groups | Diary dose | **Parameters** | | |
|---|---|---|---|---|
| | | **IL-10** (pg/ml) | **IL-6** (pg/ml) | **TFN-alfa** (pg/ml) |
| Naive | 0 | 14,2 ± 1,2 | 200,2 ± 16,2 | 8,1 ± 1,0 |
| Placebo | 1 ml/kgPV | 13,2 ± 1,1 | 188,2 ± 20,2 | 8,2 ± 1,2 |
| FOS ingredion | 257 mg/kgPV | 15,1 ± 1,0 | 220,5 ± 19,5 | 9,3 ± 1,1 |
| FOS YES | 257 mg/kgPV | 19,2 ± 0,8^{abcd} | 190,2 ± 18,5 | 3,2 ± 0,9^{abcd} |
| GOS YES | 257 mg/kgPV | 17,5 ± 2,0 | 217,2 ± 30,2 | 3,3 ± 0,8^{abcd} |
| YES MOS | 257 mg/kgPV | 18,4 ± 1,8 | 198,5 ± 25,5 | 5,9 ± 1,9 |
| Glucan MOS YES | 257 mg/kgPV | 19,3 ± 2,0 | 230,1 ± 37,5 | 6,2 ± 1,8 |
| Glucan Gold YES | 257 mg/kgPV | 25,6 ± 0,9^{abcd} | 170,9 ± 35,3 | 4,1 ± 0,7^{abcd} |
| FOS+GOS YES | 257 mg/kgPV | 30,4 ± 1,8^{abcd} | 164,8 ± 30,2 | 3,0 ± 1,0^{abcd} |
| **Composition (*)** | **257 mg/kgPV** | **33,2 ± 1,0^{abcd}** | **177,2 ± 28,5** | **3,0 ± 1,2^{abcd}** |
| Enramicine | 96 mg/kgPV | 15,3 ± 5,1 | 210,5 ± 25,5 | 8,0 ± 1,5 |
| (*) Composition, according to the invention, comprising 20% of 1,3 and 1,6 beta-glucans, 29% of GOS and 31% of FOS, mass over mass % | | | | |
| The values are expressed as the average + standard error of the average. ANOVA of a second way followed by the Bonferroni test. **^{a}p < 0,05 in comparison to naive group; ^{b}p < 0,05 in comparison to placebo group; ^{c}p < 0,05 in comparison to FOS ingredion; ^{d}p < 0,05 in comparison to ^{a}p < 0,05 in comparison to Enramicine group.** | | | | |

Table 7 indicates the effects of treatments with different isolated components, with the Composition according to the invention, or with Enramincin, on the serum levels of interleukin 10 (IL-10), IL-6 and tumor necrosis factor alpha (TNF-alpha) of male Wistar rats.

### Level 2

In phase 2, the experiment consisted of repeating the treatments of Phase 1, for 10 days, and inoculating the groups, except the so-called naive (negative control), with a high dose of *Salmonella enteritidis.* After 20 days, the mortality and the behavior of the immune system of the rats of each group were observed.

In this way, different groups of male rats were randomly marked on the tails for identification. The animals were adapted to the laboratory environment for one week before the beginning of the experiments. After a 6 h fasting period, the animals' body weight was determined and the dose of the prebiotics, the composition of this invention and Enramycin were calculated. The animals were divided into 11 (eleven) groups of 10 (ten) individuals. The animals were treated daily, by the oral route (used in the animals used as reference), with placebo (vehicle; filtered water 1 mL/kg BW) or with FOS ingredion (257 mg/kg BW), FOS Yes (257 mg/kg BW). kgPV), GOS Yes (257 mg/kgPV), Yes MOS (257 mg/kgPV), GlucanMOS (257 mg/kgPV), Glucangold Yes (77 mg/kgPV), Mixture 1 (FOS Yes + GOS Yes) (257 mg /kgPV), Composition of this invention (GlucanGold Yes + GOS Yes + FOS Yes, containing 20% of 1,3 and 1,6 beta-glucans, 31% of GOS and 29% of FOS by mass on mass %) (257 mg/kgPV) or with Enramycin (0,96 mg/kg BW). All treatments were performed for 20 days. After 10 days from the beginning of treatments, the aforementioned groups received a challenge with 1 mL (10⁸ colony forming units [cfu]/mL) of a strain of *Salmonella enterica* serovar Enteritidis (phagetype PT4), orally (gavage).

An experimental group called naive underwent sham gavage (no treatment) and was not infected with any bacterial strain. At the end of the experiments, and before euthanasia, the surviving animals were fasted for 6 hours with free access to water. Then, under anesthesia with isoflurane (2-3%), 5 mL of blood was obtained from the left jugular vein for the evaluation of biochemical parameters. After this procedure, the animals were euthanized with a supra-pharmacological dose of isoflurane (10-15%) in a saturation chamber. Immediately after euthanasia, target organs were removed for analysis.

The results shown in Table 8 show, with high statistical significance, that the group that was treated with the composition of this invention presented a balance of anti- and pro-inflammatory cytokines that allows the conclusion that they had a more modulated and activated immune system than the others. groups. The fact that it had zero mortality, as well as the group treated with the antibiotic enramycin, reinforces this conclusion.

Table 8, below, indicates the effects of treatments with different isolated components, with the Composition according to the invention, or with Enramincin, on the serum levels of interleukin 10 (IL-10), IL-6 and tumor necrosis factor alpha (TNF-alpha) from male Wistar rats infected with *Salmonella enterica.*

**TABLE 8**

| Experimental groups | Diary dose | **Deaths** | **Parameters** | | |
|---|---|---|---|---|---|
| | | | **IL-10** (pg/ml) | **IL-6** (pg/ml) | **TFN-alfa** (pg/ml) |
| Naive | 0 | 0% | 16,5 ± 1,9 | 198,2 ± 22,7 | 8,9 ± 1,1 |
| Placebo | 1 ml/kgPV | 40% | 9,2 ± 0,8^{a} | 322,1 ± 28,9^{a} | 16,1 ± 1,2^{a} |
| FOS ingredion | 257 mg/kgPV | 30% | 12,1 ± 1,8 | 268,8 ± 35,5 | 13,4 ± 1,4 |
| FOS YES | 257 mg/kgPV | 20% | 14,1 ± 0,7^{b} | 249,9 ± 40,1 | 10,6 ± 0,8^{b} |
| GOS YES | 257 mg/kgPV | 20% | 15,5 ± 0,8^{b} | 243,3 ± 45,5 | 10,1 ± 0,9^{b} |
| YES MOS | 257 mg/kgPV | 30% | 13,8 ± 1,3 | 259,9 ± 39,9 | 12,2 ± 1,8 |
| Glucan MOS YES | 257 mg/kgPV | 30% | 13,3 ± 2,0 | 245,5 ± 40,0 | 11,9 ± 1,6 |
| Glucan Gold YES | 257 mg/kgPV | 10% | 15,5 ± 0,7^{b} | 241,0 ± 55,5 | 8,0 ± 0,7^{b} |
| FOS+GOS YES | 257 mg/kgPV | 10% | 16,4 ± 1,2^{b} | 239,9 ± 38,8 | 7,4 ± 1,2^{b} |
| **Composition (*)** | **257 mg/kgPV** | **0%** | **17,5 ± 1,1^{b}** | **251,1 ± 42,2** | **8,1 ± 1,3^{b}** |
| Enramicine | 96 mg/kgPV | 0% | 16,6 ± 0,9^{b} | 249,7 ± 37,7 | 10,7 ± 1,1^{b} |
| (*) Composition, according to the invention, comprising 20% of 1,3 and 1,6 beta-glucans, 29% of GOS and 31% of FOS, mass over mass % | | | | | |
| The values are expressed as the average + standard error of the average. ANOVA of a second way followed by the Bonferroni test. **^{a}p < 0,05 in comparison to naïve group; ^{b}p < 0,05 in comparison to placebo group.** | | | | | |

### Example 2

Objective: To show the Effects of the Composition of this Invention in a Model of Obesity and Insulin Resistance in C57BU6 Mice.

For the purpose of this experiment, the composition of this invention was named Icaria.

Obesity and diabetes are part of chronic non-communicable diseases (NCDs), which have been responsible for most deaths on the planet in recent years. These diseases have a negative effect on the sleep of their patients, as well as having their condition worsened by the lack of it.

Therefore, the experiment with the supplement or product called Icária, which is a composition according to this invention, containing chelated minerals (zinc, magnesium and selenium), phytotherapic (Silymarin) and prebiotics (FOS and GOS) and Betaglucans, was intended to show that it acts as an antioxidant and neuro-endocrine-immune modulating supplement, whether in health or disease conditions.

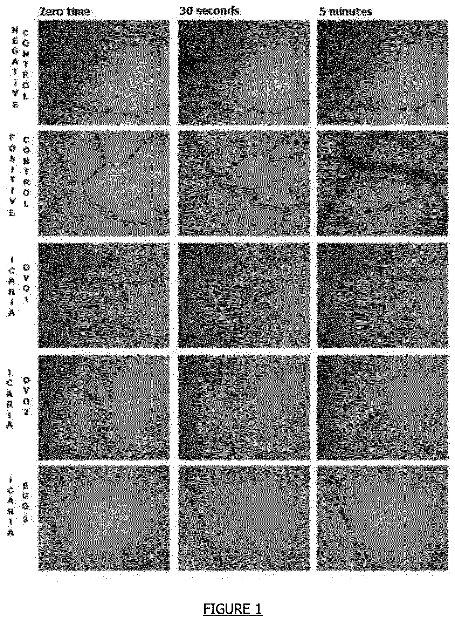

The first step was to evaluate the cytotoxicity of the composition in a chicken embryonated egg chorio-allantoic membrane (HET-CAM) assay (Figure 1). This test consisted of applying the composition, for a maximum time of 05 minutes, on the chorio-allantoic membrane and verifying if it would be able to provoke small hemorrhages, as observed in the line in line 2, in which NAOH (Hydroxy of Sodium - 1 Molar) as a positive control. And verify that this concentration was able to lyse blood vessels, resulting in hemorrhage. As we can see, the test performed in triplicate, with the Icarian composition (lines 3, 4 and 5 of the figure), behaved like the test performed only with saline (NaCl - Sodium Chloride, line 1), here our negative control, showing no hemorrhagic (cytotoxic) effect on blood vessels. Therefore, the Icarian composition proved to be safe for oral use.

Figure 1, attached, depicts a panel demonstrating the chorio-allantoic membrane assay of hen embryonated egg (HET-CAM) with Saline (NaCl) negative control, 1M Sodium Hydroxide (NaOH) positive control and Icarium composition in triplicate. Figure 1 shows that the composition of this invention did not cause any irritation or reaction on the egg embryos during the 5 minutes in which it was in contact.

In order to verify the effects of the Icarian composition under conditions that simulate health and disease, tests were carried out with C57BL/6J mice that were fed a normolipid (Regular Chow) and a high fat (HFD-High Fat Diet) diet, herein called control groups, and obese, respectively. After 10 weeks of treatment with their respective diets, the groups were divided and treated with the Icarian composition (table 9 and table 10) and with 2% carboxymethyl cellulose (carboxymethyl cellulose was used as an emulsifier for the Icarian composition, which is insoluble in water, therefore, the emulsifier was evaluated to prove that only the elements of the Icarian composition would have an effect on the tested conditions) called the Vehicle group.

Table 9 indicates the values of the actives of the Icarian composition equivalent to a supplementation for humans. Used daily for control and obese animals that received treatment via gavage for 4 weeks.

**TABLE 9**

| **Active** | **Miligrams/day (mg/day)** |
|---|---|
| Chelated zinc (16% Zn) | 80 |
| Yeast selenium (0,2% Se) | 60 |
| Chelated magnesium (10% Mg) | 140 |
| FOS (fructo-oligosaccharides) | 1.600 |
| GOS (galacto-oligosaccharides) | 1.000 |
| Silymarin | 300 |
| Yeast comprising beta-glucans derivatives | 1.000 |

To adjust the values of table 9, which is equivalent to daily values suggested for humans, minerals were based on the DRIs - Recommended Daily Intake Dosage, Silymarin, beta-glucans and prebiotics with values within the medical recommendations, there is a calculation to make the conversion to mice, respecting the animal's body area.

It is important to note that minerals are chelated to a set of amino acids, which causes the values to be calculated differently. The chelates used were di- and tripeptides and, based on experiments carried out by the manufacturer, had a bioavailability of 183% to the same minerals in the form of sulfates.

Table 10 below presents the values of the assets of the Icarian composition, converted to values supplemented to mice. The amounts were used per day in the control and obese animals that received the treatment via gavage for 4 weeks.

**TABLE 10**

| **Active** | **Miligrams/day (mg/day)** |
|---|---|
| Chelated zinc (16% Zn) | 1,1 |
| Yeast selenium (0,2% Se) | 0,4 |
| Chelated magnesium (10% Mg) | 1,3 |
| FOS (fructo-oligosaccharides) | 14,0 |
| GOS (galacto-oligosaccharides) | 9,0 |
| Silymarin | 0,9 |
| Yeast comprising beta-glucans derivatives | 6,6 |

A daily solution was made with the active ingredients of the Icaria composition, solubilized in mineral water with 2% carboxymethyl cellulose, and the amount per kilogram (kg) of animal was administered by gavage. The average volume administered to the control group was 0,3 ml and to the obese group 0,45 ml, during 4 weeks, always at the end of the day.

In order to evaluate the effects of Icarian composition on metabolic, endocrine, immunological and mitochondrial biogenesis parameters.

The weight gain of the animals treated with the vehicle and the Icarian composition, measured during the four weeks. The results of Table 11 show that the obese animals treated Icaria, although they showed lower final weight in relation to the obese Vehicle, suggest that the treatment with the composition of this invention acted in a way to prevent weight gain, even the animal consuming the diet HFD concomitant with treatment. The treated obese group had a lower BMI and a lower feed conversion efficiency (FFE) compared to the Vehicle obese group.

**TABLE 11**

| | Control | | | Obese | | | | |
|---|---|---|---|---|---|---|---|---|
| | Vehicle Average | | Icaria Average | Vehicle Average | | Icaria Average | | p |
| Normalized final weight | 3,53 | | 3,62 | 4,66 | | 4,45 | | p<0,01 |
| IMC | 0,39 | | 0,39 | 0,49 | | 0,46 | | |
| Food efficiency | 0,14 | c* | 0,77 | 0,77 | a*,b** | -0,08 | c* | p<0,01 |

| | | | | | | | | |
|---|---|---|---|---|---|---|---|---|
| Table 11 - Anthropometric values and feed efficiency of control and obese animals treated or not for the period of weeks with the Icarian composition: (a) difference in relation to Vehicle Control, (b) difference in relation to Vehicle Obese, (c) difference in relation to Control Icaria, (d) difference in relation to Obese Icaria, (N) number of animals (07-08 per group). BMI (Body Mass Index). Values expressed as mean and standard deviation (SD). *p<0,05; **p<0,01; ***p<0,001. | | | | | | | | |

As expected, the obese vehicle group presented glucose intolerance and the treatment with the Icarian composition was able to reverse glucose intolerance in the treated obese group (Table 12), reduce fasting blood glucose (Table 13) and did not interfere with the glycemic metabolism of the treated control group (Table 13).

**TABLE 12**

| Minutes | Control | | Obese | | | | |
|---|---|---|---|---|---|---|---|
| | Icaria | | Vehicle | | Icaria | | |
| | Average | *N* | Average | *N* | Average | *N* | p |
| 0 | 168 | 7 | 198 | 8 | 176 | 8 | p > 0,05 |
| 15 | 349 | 7 | 319 | 8 | 258 | 8 | c**, b* |
| 30 | 332 | 7 | 339 | 8 | 277 | 8 | c*, b* |
| 60 | 254 | 7 | 304 | 8 | 253 | 8 | p > 0,05 |
| 90 | | | 258 | 8 | 193 | 8 | c* |
| 120 | 161 | 7 | 238 | 8 | 163 | 8 | b**, c** |

| | | | | | | | |
|---|---|---|---|---|---|---|---|
| Table 12- Curve of the glucose tolerance test (GTT) of control and obese animals treated or not for a period of weeks with the Icarian composition. (c) difference in relation to Control Icaria, (b) difference in relation to Obese Vehicle, (d) difference in relation to Obese Icaria. (N) number of animals. Values expressed as mean ± standard error (SEM). *p<0,05; **p<0,01; ***p<0,001. | | | | | | | |

The evaluation of the effects of the Icarian composition on metabolism and systemic inflammation was carried out through plasma measurements of the lipid profile and plasma CRP.

Treatment with the Icarian composition of this invention reduced plasma levels of total cholesterol, triglycerides, lipoproteins (LDL, VLDL) and CRP in the Icarian control group, when compared to the vehicle control (Table 13). In addition, treatment with Icarium composition in obese animals was able to decrease the levels of total cholesterol and CRP, and increase the level of HDL-cholesterol, at levels similar to those of the control groups, as can be seen in Table 13 (Profile Lipid mg/dL).

**TABLE 13**

| Plasma | Control | | Obese | | p |
|---|---|---|---|---|---|
| | Vehicle | Icaria | Vehicle | Icaria | |
| Fast glicemia | 158 | 157 | 207 a*, c**, d* | 171 | p < 0,01 |
| PCR | 7,9 | 7,8 | 9,7 a*,c, d** | 7,8 | p < 0,01 |
| Total cholesterol | 80 | 76 | 134 a, c, d*** | 106 a**,c* | p < 0,001 |
| Triglycerides | 90 | 81 | 80 | 78 | p > 0,05 |
| HDL | 52 | 58 | 40 | 45 b* | p < 0,05 |
| LDL | 44 | 44 | 83 a, c, d* | 50 | p < 0,05 |
| VLDL | 16 | 13 | 16 | 15 | p > 0,05 |

| | | | | | |
|---|---|---|---|---|---|
| Table 13- Biochemical and inflammatory dosage of plasma of control and obese animals treated or not for a period of weeks with the Icarian composition. (a) difference in relation to Vehicle Control, (b) difference in relation to Obese Vehicle, (c) difference in relation to Control Icaria, (d) difference in relation to Obese Icaria. (N) number of animals (07-08 per group). Values expressed as mean and standard deviation (SD). CRP(C-Reactive Protein), HDL(*High-density lipoprotein),* LDL(*Low-density lipoprotein),* VLDL(*Very Low-density lipoprotein).* *p<0,05; **p<0,01; ***p<0,001. | | | | | |

The endocrine hormones cortisol (plasma) and serotonin produced in the large intestine were evaluated under the effects of the Icarian composition, as shown in Table 14. The results showed higher concentrations of plasma cortisol in the two treatments that did not receive the Icarian composition, when compared to the treatments that received it. There was also a positive correlation (r=0,91 Sperman) between cortisol and CRP within the obese vehicle group, which denotes that the condition of systemic inflammation, represented here by high plasma CRP levels, is associated with an increase in circulating cortisol. With the supply of the Icarian composition, this correlation disappeared in the treated obese animals, due to the decrease in cortisol in the Obese Icarian group, confirming the anti-inflammatory and endocrine modulating effect of the composition.

The Icarian composition also reduced plasma cortisol levels in the control group. Although serotonin is known as the hormone that regulates appetite, sleep and mood, its excess contributes to an increase in blood glucose and adipose tissue, as it is considered obesogenic. Probably, by the activation of tryptophan hydroxylase -1 (Tph1), which is related to the accumulation of fat in *in vivo models,* and to the alteration of glycemic metabolism. Serotonin is an amine derived from the amino acid tryptophan, which in addition to being produced in the brain, has most of its production in enterochromaffin cells, present in the large intestine. This pattern of high serotonin levels in obese mouse models is common, probably due to increased tryptophan intake. However, the Icarian composition seems to regulate this phenotype.

As it was expected, the serotonin produced by the intestine was in greater quantity in the obese vehicle group when compared to the vehicle control group, and the treatment with the Icarian composition was able to reduce enteroendocrine serotonin, thus confirming the modulation of the composition already at the intestinal level by the modulation of a hormone that, when produced in excess by the intestine, is associated with obesity, as can be seen in Table 14 (Endocrine Hormones).

**TABLE 14**

| | Unit | Control | | | Obese | | | p |
|---|---|---|---|---|---|---|---|---|
| | | Vehicle | | Icaria | Vehicle | | Icaria | |
| | | Average | | Average | Average | | Average | |
| Cortisol (plasma) | pg/ml | 59,9 | c,d* | 57,3 | 58,7 | d* | 57,1 | p < 0,05 |
| Serotonin (large intestine) | pmol/ml | 11,5 | | 13,5 | 15,3 | a*,d* | 12,6 | p < 0,05 |

| | | | | | | | | |
|---|---|---|---|---|---|---|---|---|
| Table 14 - Evaluation of circulating cortisol and serotonin produced by enterochromaffin cells of the large intestine by ELISA of control and obese animals treated or not for a period of weeks with the Icarian composition. (a) difference in relation to Vehicle Control, (b) difference in relation to Obese Vehicle, (c) difference in relation to Control Icaria, (d) difference in relation to Obese Icaria. (N) number of animals (07-08 per group). Values expressed as mean and standard deviation (SD). *p<0,05; **p<0,01; ***p<0,001. | | | | | | | | |

Table 14 shows that the serotonin concentration in the Vehicle Obese group of animals was at a higher level than in the Control group. However, when the Obese group was treated with the Icarian composition, this concentration was equal to that of the Control group and decreased in relation to its respective Vehicle group. Although a significant increase was not observed between the Control groups, the Icaria supplement was able to increase the serotonin level in the Control Icaria group, suggesting an improvement in the mood and sleep of eutrophic animals.

Treatment with the Icarian composition was also able to modulate the lipid content of the liver. Animals that consumed the HFD diet presented a picture of hepatic steatosis. With the treatment with the Icarian composition, a reduction in the levels of total cholesterol, triglycerides and VLDL was observed in the animals of both obese and control groups. Likewise, a significant increase in HDL was observed in both groups when treated with the Icarian composition, as shown in Table 15 - Liver Lipids (mg/g). The reduction in hepatic fat levels was evidenced by histological analysis of the liver (H&E *staining),* both in control and treated obese animals. In addition, a decrease in the accumulated fat droplets was observed, mainly around the hepatic vein of the lobules of the treated obese animals.

**TABLE 15**

| Liver | Control | | Obese | | p |
|---|---|---|---|---|---|
| | Vehicle | Icaria | Vehicle | Icaria | |
| | Average | Average | Average | Average | |
| Total cholesterol | 5,5 | 4,6 | 7,3 | 4,2 | p < 0,05 |
| Triglycerides | 4,0 | 1,6 | 2,2 | 1,0 | p < 0,05 |
| HDL | 3,0 | 3,1 | 1,6 | 3,0 | p > 0,05 |
| VLDL | 0,3 | 0,2 | 0,8 | 0,4 | p < 0,05 |

| | | | | | |
|---|---|---|---|---|---|
| Table 15- Assessment of the lipid profile, by means of alcoholic extraction from the liver tissue of control and obese animals treated or not for a period of weeks with the Icarian composition. (a) difference in relation to Vehicle Control, (b) difference in relation to Obese Vehicle, (c) difference in relation to Control Icaria, (d) difference in relation to Obese Icaria. (N) number of animals (07-08 animals per group). Values expressed as mean and standard deviation (SD). *p<0,05; **p<0,01; ***p<0,001. | | | | | |

The inflammation and oxidative stress that accompany not only obesity, but chronic diseases such as diabetes, cancer, inflammatory bowel diseases, and why not mention viral infections such as SARS-Cov2, which have in common the activation of transcription factors of inflammatory cytokines. Thus, NFkappa-B (Nuclear Factor KappaB) was evaluated, which is one of the main regulatory factors of cellular homeostasis, and much better known for being involved in the production of pro-inflammatory factors and cytokines. Currently the term " *cytokine storm* " has become common in the medical community and among lay people, and it is exactly this factor that is directly involved in this process. Table 16 shows that the treatment using the Icarian composition provided a reduction in the levels of NF-kappaB in the liver tissue of the control and obese animals, confirming the anti-inflammatory effect on the tissue, which had already been manifested by the reduction of plasma CRP., since this protein is produced in the liver.

In addition to the anti-inflammatory effect, the Icarian composition has shown promise in mitochondrial modulation, which can help in the prevention and treatment of mitochondriopathies. Today, mitochondria are considered a focus for the development of compounds that act on this cellular organelle. Oxidative stress originates from the imbalance caused by the excessive generation of reactive species and the lack of substances that form the endogenous and exogenous antioxidant systems. This condition is present in all diseases and its place of origin is in the mitochondria, and may also occur in the rough endoplasmic reticulum.

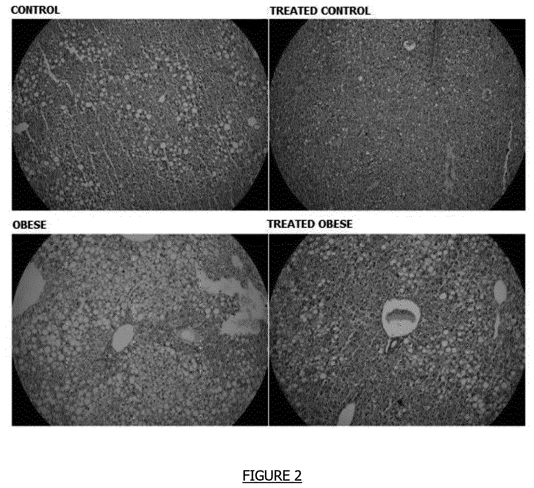

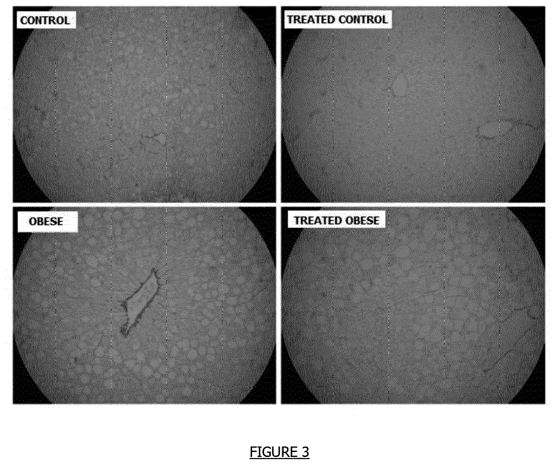

To evaluate the effect of the treatment with the Icarian composition on proteins involved in regulatory pathways of cellular homeostasis and mitochondrial biogenesis, the protein expressions of NRF1 (Nuclear Respiratory Factor 1) and TFAM (Mitochondrial Activation Factor) were analyzed. The results shown in Table 16 show that the composition was able to significantly and expressively increase the levels of NRF1 in the treated control group, when compared to its vehicle control, as well as in the obese animal groups. This suggests a modulating effect on the liver tissue of the control group, which, according to histological analysis, showed easily visible improvements, in Figures 2 and 3 attached, in metabolic and inflammatory parameters. In the treated obese group, an increase in TFAM was observed, indicating that there was a modulation of this factor, which stimulates mitochondrial biogenesis, which increases the oxidative capacity of the organelle and reduces excess fat in the liver. As evidenced by the decrease in hepatic steatosis and the surrounding and tissue lipids and fat, as well as the reduction of fibrosis, which can be seen in attached figures 2 and 3. Figure 2 deals with the effect of the composition of this invention showing that there was a significant decrease in hepatic steatosis (fat accumulation) in the liver tissues of the animals in the Control and Obese groups, when treated with the composition of this invention. Figure 3 demonstrates the anti-fibrogenic effect of the composition of this invention on the liver tissue of Control and Obese animals, treated or not. The tissue was treated with picrosirius dye, which highlights the fibrosis in red.

**TABLE 16**

| Proteins | Control | | | | Obese | | | p |
|---|---|---|---|---|---|---|---|---|
| | Vehicle | | Icaria | | Vehicle | | Icaria | |
| | Average | | Average | | Average | | Average | |
| Nfk-B | 0,72 | c,d*** | 0,14 | | 1,03 | a*,c***,d*** | 0,15 | p<0,001 |
| TFAM | 0,37 | | 0,42 | a**,d* | 0,59 | | 0,71 | |
| NRF1 | 0,58 | | 1,01 | a*,b,d*** | 0,37 | | 0,29 | |

| | | | | | | | | |
|---|---|---|---|---|---|---|---|---|
| Table 16. Evaluation of inflammatory transcription factors and those involved in mitochondrial biogenesis of control and obese animals, treated or not with the Icarian composition. (a) difference in relation to Vehicle Control, (b) difference in relation to Obese Vehicle, (c) difference in relation to Control Icaria, (d) difference in relation to Obese Icaria. (N) number of animals (07-08 animals per group). Values expressed as mean and standard deviation (SD). *p<0,05; **p<0,01; ***p<0,001. | | | | | | | | |

Corroborating the increase in NRF1 in the liver tissue, the animals treated with the Icarian composition in the control group showed a gain in lean mass (muscle), as shown in Table 17. NRF1, in addition to acting on mitochondrial biogenesis, is also associated with pathways of protein synthesis activation, suggesting that the Icarian composition positively modulated tissue protein production, not only in the liver, but also in muscle tissue.

**TABLE 17**

| Portions | Control | | | | Obese | | | | p |
|---|---|---|---|---|---|---|---|---|---|
| | Vehicle | | Icaria | | Vehicle | | Icaria | | |
| | Average | | Average | | Average | | Average | | |
| Lean mass | 19,4 | b,d*** | 21,7 | a* | 23,8 | | 23,4 | | p<0,001 |
| Fat | 6,1 | | 6,1 | | 12,5 | a,c*** | 12,0 | a*** | p<0,001 |

| | | | | | | | | | |
|---|---|---|---|---|---|---|---|---|---|
| Table 17 - Evaluation of body composition through magnetic resonance imaging of control and obese animals treated or not for a period of weeks with the Icarian composition. (a) difference in relation to Vehicle Control, (b) difference in relation to Obese Vehicle, (c) difference in relation to Control Icaria, (d) difference in relation to Obese Icaria. (N) number of animals (07-08 animals per group). Values expressed as mean and standard deviation (SD). *p<0,05; **p<0,01 ***p<0,001. | | | | | | | | | |

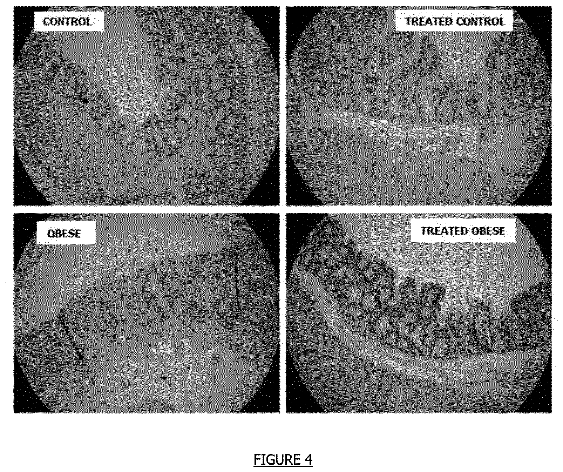

The morphological modulation of the liver, observed in tables 12, 13 and 15, as well as in figures 2 and 3, was due to the synergistic effect of the composition, and so far no analysis using beta-glucans derived from *S*. *cerevisiae* associated silymarin, silymarin and prebiotics had been performed, reinforcing the novelty of the present invention, and its metabolic and protective effects on hepatic steatosis. These prebiotics also provided a significant increase in the population of bifidobacteria, whose higher production of butyric acid allowed the filling of goblet cells with mucus, as can be seen in Figure 4 attached. Figure 4 is a section of the Large Intestine of C57BU6 mice, at the time of insertion of the Ileum, where the effect of the composition of this invention on the filling of the goblet cells of the Control and Obese groups, treated or not, can be observed. Two-way ANOVA analyzes were used, with post Bonferroni test and *Student's t test.*

As a partial conclusion, the Icarian composition showed potential to improve sleep quality through the modulation of lipid and carbohydrate metabolism, as it modulated glycemic and lipid levels, being also a promising treatment alternative in diseases involving this system such as obesity, diabetes., anxiety.

Another effect produced by the Icarian composition, which has great potential to improve sleep quality, was that it has shown to be an endocrine system regulator, since it was able to modulate plasma cortisol levels. In addition to showing that it is a regulator of the intestinal production of serotonin.

### Example 3

Objective: To show the Effects of the Composition of this Invention on Sleep Quality, Blood Lipid Profile and Peristalsis in Humans (preliminary tests).

There is a lot of evidence that the use of the composition of this invention promotes an increase in the production of brain and intestinal serotonin, as well as helps to regulate its release in adequate amounts to the body, in order to improve sleep quality, increasing sleep duration. deep, mainly its NREM 3 and 4 (SWS) stages.

Approximately 90% of serotonin production takes place in the intestines. Its action is a vasoconstrictor on the smooth muscles of the intestines, so that the greater its production, the greater the peristalsis tends to be. Which can be observed by the increase in the number of bowel movements per day.

Serotonin also contributes to increasing the proportion of NREM 3 and 4 sleep, which is the stage of sleep where cortisol is eliminated and when growth hormone production occurs, which stimulates mitochondrial biogenesis.

The experiment presented below was carried out to evaluate the magnitude of the effects of the composition of this invention on bowel movements, liver modulation (blood lipid profile) and sleep quality of patients using it. For this purpose, 18 volunteers (11 women and 7 men) were selected, aged between 37 and 78 years, with an average weight of 74 kg of live weight, who agreed to participate in the experiment for 6 months, recording the number of bowel movements and measuring the sleep quality, in addition to noting other changes in health status.

A composition, according to this invention, was used to be consumed in capsules containing 435 mg each, according to Table 18 below:

**TABLE 18**

| **Content per composition capsules** | | | | |
|---|---|---|---|---|
| Raw materials | Purity | Total mass (1) | Pure mass (2) | (2)/(1) |
| FOS ingredion | 95% | 120 mg | 114 mg | 26,2% |
| YES GOS | 70% | 120 mg | 84 mg | 19,3% |
| GlucanGOLD YES (*) | 60% | 60 mg | 36 mg | 8,3% |
| YES Zinc | 16% | 15 mg | | |
| YES Selenium | 0,2% | 10 mg | | |
| YES Magnesium | 10% | 30 mg | | |
| Silymarin | 100% | 40 mg | 40 mg | 9,2% |
| Tyrosyl | 100% | 40 mg | | |
| Total | | 435 mg | 274 mg | |

| | | | | |
|---|---|---|---|---|
| (*) GlucanGOLD YES is the source of 1,3 and 1,6 beta-glucans with 60% purity | | | | |

Despite the difference in live weight between the volunteers, for practical reasons, the same daily dose was used for all volunteers, 4 capsules/volunteer, two taken at dawn and two at dusk. For the purposes of this invention, this corresponded to a daily intake of 1096 mg/day (274 mg/capsule x 4 = 1096 mg/day) per volunteer with an average weight of 73,6 kg live weight, i.e. 14,9 mg/kgPV/day. Which is within the range considered ideal and safe for human consumption, according to this invention.

The experiment started with a period of 60 days of adaptation and self-knowledge of the volunteers, before they started to ingest the capsules, with the composition of this invention. During this period, each volunteer received a *MiBand* 3 bracelet, manufactured by Xiaomi, and a table to fill in the daily information on sleep (collected by the respective bracelets) and on the number of bowel movements. During this period, blood samples were also collected from the volunteers for analysis of total cholesterol, HDL and triglycerides. The last 30 days of information from each volunteer were considered as the control information, called M -1 in Tables 19, 20 and 21 presented below.

**TABLE 19**

| Months | **Hours of sleep per night** | | | | | | |
|---|---|---|---|---|---|---|---|
| | **Profound** | | | **Total** | | | |
| | Min | Aver. (1) | Max | Min | Aver. (1) | Max | (1)/(2) |
| **M -1** | 01:13 | 01:32 | 02:15 | 06:12 | 06:56 | 07:23 | 22% |
| M 1 | 01:15 | 01:30 | 02:10 | 06:05 | 06:54 | 07:20 | 22% |
| M2 | 01:23 | 01:35 | 02:17 | 06:15 | 06:58 | 07:30 | 23% |
| M 3 | 01:20 | 01:46 | 02:25 | 06:25 | 06:59 | 07:22 | 25% |
| M 4 | 01:26 | 01:49 | 02:29 | 06:12 | 07:04 | 07:35 | 26% |
| M 5 | 01:30 | 01:51 | 02:30 | 06:01 | 07:07 | 07:32 | 26% |
| **M 6** | 01:31 | 01:52 | 02:32 | 06:12 | 07:08 | 07:38 | 26% |
| ***Increase*** | ***25%*** | ***22%*** | ***13%*** | ***0%*** | ***3%*** | ***3%*** | ***18%*** |

Table 19 shows the average number of hours of deep sleep and total sleep of the volunteers, measured by the MiBand 3 bracelets. The month called M -1 corresponds to the last 30 days of adaptation and self-knowledge of each volunteer and considered the control, for of comparison. The remaining months (from M1 to M6) are the period of ingestion of capsules containing the composition of this invention.

The results in Table 19 show that the ingestion of the capsules increased the amount of deep sleep in the volunteers. On average this increase was 22%. As there was no significant increase in the total number of hours of sleep during the period of the experiment, it can be concluded that the ingestion of the capsules of the composition of this invention provided an average increase of 18% in the proportion of deep sleep of the volunteers, after 6 months of ingestion. of the capsules.

It is also interesting to note that the effect of composition on deep sleep gradually increased over the 6 months. However, in a faster way until the fourth month, as shown in Table 20.

**TABLE 20**

| Months | Sleep quality (*) | | | **Better than the Benchmark (**)** | | |
|---|---|---|---|---|---|---|
| | Min | Aver. | Max | Min | Aver. | Max |
| **M -1** | 69 | 74 | 89 | 32% | 42% | 84% |
| M 1 | 68 | 74 | 88 | 30% | 42% | 81% |
| M 2 | 71 | 75 | 91 | 35% | 44% | 89% |
| M 3 | 72 | 77 | 92 | 37% | 49% | 91% |
| M 4 | 74 | 79 | 93 | 42% | 54% | 93% |
| M 5 | 72 | 80 | 93 | 37% | 57% | 93% |
| **M 6** | 74 | 81 | 92 | 42% | 60% | 91% |
| ***Increase*** | ***7%*** | ***9%*** | ***3%*** | ***31%*** | ***43%*** | ***8%*** |

Table 20 shows the evolution of the volunteers' sleep quality. The month called M -1 corresponds to the last 30 days of adaptation and self-knowledge of each volunteer and considered the control, for comparison purposes. The remaining months (from M1 to M6) are the period of ingestion of capsules containing the composition of this invention.

Sleep quality is a score by which the *MiBand* 3 bracelet app, used by volunteers, evaluates each one's sleep each night. This score depends on the amount of deep sleep and total sleep, as well as the distribution of deep sleep throughout the night and its duration in each cycle. The results show that, over 6 months of ingestion of the capsules containing the composition of this invention, on average, the volunteers improved by 7 points in the evaluation, that is, a 9% increase in the score.

The wristband app also compares sleep assessment scores with the average of wristband wearers around the world, which we call the *Benchmark.* According to this comparison, on average in month M -1 (last 30 days before the start of capsule ingestion) the volunteers obtained an average score equal to 74 points, which corresponds to better sleep than 42% of global users of MiBand3 bracelets.. After 6 months of taking the capsules, containing the composition of this invention, the average score of the volunteers rose to 81 points, which the Benchmark indicates that the sleep quality of the volunteers was better than that of 60% of global users of the bracelets. Which is a significant 43% improvement.

**TABLE 21**

| **Evacuations per day** | | | |
|---|---|---|---|
| Months | Minimum | Average | Maximum |
| **M -1** | 0,73 | 0,95 | 1,53 |
| M 1 | 0,98 | 1,32 | 1,89 |
| M 2 | 1,02 | 1,34 | 1,93 |
| M 3 | 0,97 | 1,32 | 1,89 |
| M 4 | 0,97 | 1,31 | 1,97 |
| M 5 | 0,95 | 1,30 | 1,93 |
| **M 6** | 0,99 | 1,32 | 1,96 |
| **Increase** | **36%** | **39%** | **28%** |

Table 21 shows the volunteers' average daily bowel movements over each month. The month called M -1 corresponds to the last 30 days of adaptation and self-knowledge of each volunteer and considered the control, for comparison purposes. The remaining months (from M1 to M6) are the period of ingestion of capsules containing the composition of this invention.

Table 21 shows that there was an increase of, on average, 39% in the number of daily bowel movements by the volunteers, after 6 months of ingestion of the capsules containing the composition of this invention. This effect is attributed to the greater production of serotonin in the intestines of the volunteers, probably due to a greater population of lactobacilli and bifidobacteria provided by the capsules. Probably, this larger population of these bacteria led to a greater availability of butyric acid to the enterochromaffin cells of the intestinal endothelium, which led them to produce greater amounts of serotonin, which increases peristalsis and contributes to increasing the duration of deep sleep, especially the stage NREM 3 and 4.

At the end of the sixth month, a new blood sample was collected from the volunteers for analysis of total cholesterol, HDL and triglycerides. The results, comparing the concentrations of these lipids in the blood, are shown in Table 22.

**TABLE 22**

| Meses | Total Cholesterol | | | HDL | | | Triglycerides | | |
|---|---|---|---|---|---|---|---|---|---|
| | Min | Aver. | Max | Min | Aver. | Max | Min | Aver. | Max |
| M -1 | 131 | 196 | 223 | 44 | 53 | 61 | 82 | 142 | 163 |
| M 6 | 114 | 188 | 197 | 45 | 57 | 76 | 72 | 109 | 128 |
| *Difference* | *-13%* | ***-4%*** | *-12%* | *2%* | *8%* | *25%* | *-12%* | ***-23%*** | *-21%* |

The results shown in Table 22 show that, after six months of consumption of capsules containing the composition of this invention, there was a significant improvement in the mean profile of lipids in the blood of the volunteers, which indicates that a beneficial modulation of the liver occurred, whose consequences must have influenced the improvement in sleep quality observed.

Over the six months of testing each volunteer, there were several observations about changes in their health status, some of which are listed below:
- more malleable and lubricated stools;
- suggestion (low incidence of flu, and with a reduction in the duration of flu symptoms) they almost didn't get flu and when they did they were weak, passing in 2 to 3 days.
- growth and strengthening of nails and hair;
- faster metabolization of alcohol;
- a volunteer, who has been diabetic for 5 years, observed hypoglycemia spikes when ingesting the capsules together with diabetes control drugs. Thus, he began to ingest the capsules at lunch and dinner. Meanwhile, medicines for diabetes began to be taken at dawn. It worked, but his blood sugar levels over the 6 months were lower than before.

## Claims

1. Regulatory composition of the neuro-immune-endocrine system, **characterized by** comprising the following components:
- 1,3 and 1,6 beta-glucans;
- Silymarin (*Silybum marianum*)*,* or its natural or synthetic flavonolignans or flavonoids; and
- one or more prebiotics with a bifidogenic effect.

2. Regulating composition of the neuro-immune-endocrine system, according to claim 1, **characterized because** the 1,3 and 1,6 beta-glucans have an average particle diameter smaller than 100 microns.

3. Regulating composition of the neuro-immune-endocrine system, according to claim 1, **characterized by** comprising one or more prebiotics with a bifidogenic effect, which can be chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and/or Mannan-oligosaccharide (MOS) and/or glyco-oligosaccharides, their subspecies or subtypes or mixtures.

4. Regulating composition of the neuro-immune-endocrine system, according to any one of claims 1 to 3, **characterized because** Silymarin is used through leaves and seeds that can be transformed into dry powder or standardized dry extract, comprising flavonoids and flavonolignans.

5. Regulating composition of the neuro-immune-endocrine system, according to claim 1 or 4, **characterized in that** the flavonolignans may be chosen from the group comprising: silybin A, silybin B, isosilybin A, isosylbin B, silycristin A, silycristin B, isosylcristin A, dehydrosylristin A, silydianin, dehydrosilidianin and silymarin.

6. Regulatory composition of the neuro-immune-endocrine system, according to claim 3, **characterized by** comprising the following components:
- 1,3 and 1,6 beta-glucans, from the cell wall of yeasts of the *Saccharomyces* and/or *Candida genera;*
- Silymarin (*Silybum marianum*)*,* or its natural or synthetic flavonolignans or flavonoids; and
- one or more prebiotics with a bifidogenic effect chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and /or Mannan-oligosaccharide (MOS) and/or glyco-oligosaccharides, their subspecies or subtypes, and/or other prebiotics with bifidogenic action.

7. Regulating composition of the neuro-immune-endocrine system, according to any one of the preceding claims, **characterized because** the 1,3 and 1,6 Betaglucans come from the cell wall of yeasts of the *Saccharomyces* and/or *Candida genera,* with an average particle diameter of less than 100 microns.

8. Regulating composition of the neuro-immune-endocrine system, according to any one of the preceding claims, **characterized by** comprising the following components:
- 1,3 and 1,6 beta-glucans, from the cell wall of yeasts of the *Saccharomyces* and/or *Candida genera,* with an average particle diameter of less than 100 microns;
- Silymarin (*Silybum marianum*)*,* or its natural or synthetic flavonolignans or flavonoids; and
- one or more prebiotics with a bifidogenic effect, which may be chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and/or Mannan-oligosaccharide (MOS) and/or glyco-oligosaccharides, their subspecies or subtypes, and/or other prebiotics with bifidogenic action.

9. Regulating composition of the neuro-immune-endocrine system, according to any one of the preceding claims, **characterized because** it can still contain mineral micronutrients in its inorganic, organic or chelated form.

10. Regulating composition of the neuro-immune-endocrine system, according to any one of claims 1 or 6 or 8 or 9, **characterized by** comprising the following components:
- 1,3 and 1,6 beta-glucans, from the cell wall of yeasts of the *Saccharomyces* and/or *Candida genera,* with an average particle diameter of less than 100 microns;
- Silymarin (*Silybum marianum*)*,* or its natural or synthetic flavonolignans or flavonoids;
- one or more prebiotics with a bifidogenic effect chosen from the group comprising: Fructo-oligosaccharides (FOS) and/or Galacto-oligosaccharides (GOS) and/or Inulin and/or polydextrose and/or lactulose and/or Xylo-oligosaccharides (XOS) and /or Mannan-oligosaccharide (MOS) and/or glyco-oligosaccharides, their subspecies or subtypes, and/or other prebiotics with bifidogenic action;
- minerals: Zinc and/or Magnesium and/or Selenium, in their inorganic forms or associated with organic molecules.

11. Regulatory composition of the neuro-immune-endocrine system, according to claim 1, **characterized as** it comprises in pure mass in relation to the total mass of the composition: 2% to 10% of 1,3 and 1,6 Betaglucans, 38% to 70% of Prebiotics with bifidogenic effect and 4% to 11% of Silymarin.

12. Regulating composition of the neuro-immune-endocrine system, according to claim 11, **characterized as** it comprises in pure mass in relation to the total mass of the composition: 3% to 8% of 1,3 and 1,6 Betaglucans, 43% to 63% of Prebiotics with bifidogenic effect and 6% to 10% of Silymarin.

13. Regulating composition of the neuro-immune-endocrine system, according to claim 1, **characterized as** it comprises a ratio of 1,3 and 1,6 beta-glucans: Bifidogenic Prebiotics: Silymarin, in pure mass in relation to the total mass of the composition, ranging from: 0,7 to 1,3 of 1,3 and 1,6 beta-glucans, 6,5 to 12,0 of Bifidogenic Prebiotics and 0,8 to 1,4 of Silymarin.

14. Regulatory composition of the neuro-immune-endocrine system, according to claim 13, **characterized as** it comprises a ratio of 1,3 and 1,6 beta-glucans: Bifidogenic Prebiotics: Silymarin, in pure mass in relation to the total mass of the composition, ranging from: 0,9 to 1,1 of 1,3 and 1,6 beta-glucans, 7,5 to 10,5 of Bifidogenic Prebiotics and 1,0 to 1,3 of Silymarin.

15. Regulating composition of the neuro-immune-endocrine system, according to claim 13 or 14, **characterized as** it comprises a ratio of 1,3 and 1,6 Betaglucans:Bifidogenic Prebiotics:Silymarin, in pure mass in relation to the total mass of the composition, according to the ratio of 1,0:8,9:1,2 when expressed in whole numbers.
